(19) Europäisches Patentamt
European Patent Office
Office européen des brevets

(11) EP 3 662 826 A1

(12) EUROPEAN PATENT APPLICATION
published in accordance with Art. 153(4) EPC

(43) Date of publication:
10.06.2020 Bulletin 2020/24

(51) Int Cl.:
A61B 5/0476 (2006.01)    A61B 5/0484 (2006.01)
A61B 10/00 (2006.01)

(21) Application number: 18842034.3

(22) Date of filing: 30.07.2018

(86) International application number:
PCT/JP2018/028491

(87) International publication number:
WO 2019/026853 (07.02.2019 Gazette 2019/06)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 31.07.2017 JP 2017148350

(71) Applicants:
• Osaka University
Suita-shi, Osaka 565-0871 (JP)
• Hiroshima City University
Hiroshima-shi, Hiroshima 731-3194 (JP)

(72) Inventors:
• NAKAE Aya
Suita-shi
Osaka 565-0871 (JP)
• ISHIMITSU Shunsuke
Hiroshima-shi
Hiroshima 731-3194 (JP)
• TANAKA Daisuke
Hiroshima-shi
Hiroshima 731-3194 (JP)

(74) Representative: Sackin, Robert
Reddie & Grose LLP
The White Chapel Building
10 Whitechapel High Street
London E1 8QS (GB)

(54) **APPLICATION OF REAL SIGNAL TIME VARIATION WAVELET ANALYSIS**

(57) The objective of the present invention is to provide a technique which enables analysis to be performed in real-time without taking a control or the like, by performing wavelet transformation at the same time, without pre-processing of a signal. The present invention provides a pain estimating method and device with which pain of an estimation target can be objectively and accurately estimated, and with which the quality and quantity thereof can be classified simply. The present invention provides a method for processing a signal of a target in response to a stimulus, and includes: a) a step of obtaining from the target a signal in response to a stimulus; (b) a step of subjecting the signal to cross correlation processing using part or all of the signal; and (c) a step of calculating a feature quantity of the signal and a coefficient correlated to the stimulus from the processing results obtained in b).

FIG.18

**Description**

[Technical Field]

The present invention relates to application of cross-correlation processing such as real signal time variation wavelet analysis. The present invention specifically relates to a technology for determining pain by analyzing brainwaves using real signal time variation wavelet analysis.

[Background Art]

While quantification of pain such as Pain Vision is known as a technology for measuring pain, the degree of objectivity is low because such a technology is quantification based on data obtained from an object reporting subjective sensation by pressing a button.

A technology for measuring brain activity from brainwaves using evoked potential is known. The development thereof as means for finding a neurological disorder is ongoing. However, the magnitude of pain is not measured.

Meanwhile, wavelet analysis is often utilized in signal processing. Application thereof to biological signals or the like have also expanded. Wavelet analysis requires comparison with a control, so that application thereof in a situation where it is difficult to measure a control from an object is challenging.

In a routine diagnosis, alleviation of pain with an analgesic is often required. However, the effect of analgesics varies among individuals, so that the inventors have not been able to define a standard. These differences are observed due to the health condition, sex, and level of anxiety of individuals. Therefore, it is difficult to determine the drug dosage that requires an analgesic in advance. Currently, objective evaluation standards for pain have yet to be defined. Physicians determine the correct dosage based on their own evaluation of the pain level of the subject. Therefore, in some cases, a subject can be administered an unsuitable amount of analgesic. The excessive amount can result in severe complications or drug dependency. From this viewpoint, objective evaluation of pain for determining the amount of analgesic required for each subject can contribute to healthy living. Visual Analogue Scale (VAS) is a subjective evaluation method that is useful for diagnosis of pain (Non Patent Literature 1). The simplest VAS is a straight line extending in the direction of left and right with a certain length, generally 100 mm. The ends are defined as the limit values, oriented from left (worst) to right (best). Subjects mark a point on the line representing their own understanding of their own condition. There is another evaluation method for measuring the intensity of pain using electrical stimulation (Non Patent Literature 2). However, subjective description of a subject is still required. Brain function analysis using magnetic resonance imaging (MRI) is an objective evaluation method (Non Patent Literature 3), which is an invasive method that constrains a subject at an enclosed location over about 30 minutes. Since MRI requires a large-scale device, it is difficult to routinely use MRI. MRI also requires a technician who can operate an apparatus.

[Citation List]

[Non Patent Literature]

[NPL 1] N. Hirakawa, "Evaluation Scale of Pain", Anesthesia 21 Century Vol.13, No. 2-40, pp 4-11, 2011
[NPL 2] H. Arita, "Pain VisionTM", Anesthesia 21 Century Vol. 13, No.2-40, pp 11-15, 2011
[NPL 3] H. Fukuyama, M.D., Ph.D., "Tips on the functional brain imaging analysis", The Journal of Japan Society for Cognitive Neuroscience, Vol 12, No.3, 2010

[Summary of Invention]

[Solution to Problem]

The present invention provides a methodology that can analyze in real time without using a control or the like by simultaneous cross-correlation processing (e.g., wavelet transform) without pre-processing of signals. In one embodiment, the inventors found that pain stimulation can be more accurately differentiated with a cross-correlation (e.g., autocorrelation) feature by convoluting a real signal from creating and standardizing a time variation wavelet for a brainwave feature (amplitude, frequency power, or the like) used in differentiation analysis of pain levels into the original brainwave data that was standardized and generating the cross-correlation feature. An abnormal event such as pain can be objectively estimated based on analysis of signals such as biological signals.

More specifically, pain used in a specific example of cross-correlation processing is an unpleasant sensation transmitted to the brain by sensory nerves. The dose of a drug or analgesic is determined by the pain level. However, an objective standard for the evaluation of pain has not been established. Therefore, there is a risk of administering an unsuitable dose of analgesic to a subject. Thus, the inventors propose a method of estimating pain of a subject using a conventional device based on biological signals, primarily brainwaves (EEG). Specifically, the inventors extracted a characteristic of pain by using wavelet transform and the ratio (LF/HF) between a low frequency component and a high frequency component. Furthermore, the inventors proposed a novel wavelet analysis focusing on self-similarity of biological signals using the mother wavelet of EEG. The inventors monitored EEG, skin conductance, peripheral blood capillary oxygen saturation ($SpO_2$), and pulse waves as biological signals induced by temperature stimulation. In a specific example of cross-correlation processing, the stimulation exemplified in the Examples was alternatingly applied to 20 or more healthy subjects. When distressing pain was applied, a feature obtained from the $\alpha$ wave band of EEG weakened. It is understood that this is because of the breakdown in self-similarity due to instability of EEG waveforms in view of pain.

In a specific example of cross-correlation processing, the inventors studied an objective method for evaluating pain levels. The method of the invention detects pain of a subject based on a biological signal primary derived from EEG by using a conventional device. The inventors also measured the degree of oxygen saturation by $SpO_2$, pulse waves, and skin conductance, in addition to monitoring of EEG. The inventors also studied $\alpha$ waves in EEG analysis. The method of the invention also studied self-similarity of EEG by using wavelet transform. Generally, when evaluating a fractal dimension, almost all biological signals have a certain degree of self-similarity, by which the inventors can distinguish a healthy state and a state accompanying distress. Since this experiment was invasive to humans, the experiment was conducted in accordance with ethical guidelines.

Therefore, the present invention provides the following.

(1) A method of processing a signal of an object in response to stimulation, comprising:

a) obtaining a signal in response to stimulation from the object;
b) applying cross-correlation processing to the signal using a part or all of the signal; and
c) calculating a feature of the signal and a coefficient associated with the stimulation from a result of processing obtained in b).

(2) The method of item 1, wherein the cross-correlation processing comprises autocorrelation processing.
(3) The method of item 1 or 2, wherein the correlation processing comprises finding self-similarity for each time.
(4) The method of any one of items 1 to 3, wherein the signal does not have self-similarity or has a missing portion.
(5) The method of any one of items 1 to 4, wherein the signal is a biological signal.
(6) The method of any one of items 1 to 5, wherein the biological signal is a brain signal.
(7) The method of items 1 to 6, wherein the correlation processing comprises real signal wavelet transform.
(8) The method of any one of items 1 to 7, further comprising subjecting the wavelet transformed signal to convolution processing into the signal data before the transform in step b) .
(9) The method of any one of items 1 to 8, wherein the correlation processing comprises creating a time variation wavelet, normalization of the signal, and convolution of the normalized signal.
(10) The method of items 1 to 9, wherein the correlation processing comprises performing instantaneous correlation analysis.
(11) The method of any one of items 1 to 10, wherein step b) comprises:

b-1) sampling a segment of the signal;
b-2) generating a mother wavelet from the signal;
b-3) optionally analyzing a correlation with the signal by extending and contracting the mother wavelet; and
b-4) repeating b-1) to b-3) until a portion necessary for analysis of the signal is analyzed.

(12) The method of any one of items 1 to 11, wherein the signal comprises at least one signal calculated in a frequency band of $\delta$, $\theta$, $\alpha$, $\beta$, and $\gamma$ and four electrodes.
(13) The method of any one of items 1 to 12, wherein the feature and the coefficient are associated so that the level of the stimulation can be differentiated in the best manner by sigmoid fitting or a multiple regression model.
(14) A method of analyzing an object, comprising:
analyzing a reaction to the stimulation of the object using the feature and the coefficient obtained by the method of any one of items 1 to 13.
(15) The method of item 14, wherein the reaction comprises pain.
(16) An apparatus for processing a signal of an object, comprising:

A) a signal obtaining unit for obtaining a signal from an object;
B) a processing unit for applying cross-correlation processing to the signal using a part or all of the signal; and
C) a calculation unit for calculating a feature of the signal and a coefficient associated with the stimulation from a result of processing obtained in B).
(16A) The apparatus of item 16, further comprising a characteristic of any one or more of items 1 to 15.

(17) An apparatus for analyzing an object, comprising:

A) a signal obtaining unit for obtaining a signal from an object;
B) a processing unit for applying cross-correlation processing to the signal using a part or all of the signal;
C) a calculation unit for calculating a feature of the signal and a coefficient associated with the stimulation from a result of processing obtained in B); and
D) an analysis unit for analyzing a characteristic of the object using the feature and the coefficient.
(17A) The apparatus of item 17, further comprising a characteristic of any one or more of items 1 to 15.

(18) A program for making a computer perform a method of processing a signal of an object in response to stimulation, the method comprising:

a) obtaining a signal in response to stimulation from the object;
b) applying cross-correlation processing to the signal using a part or all of the signal; and
c) calculating a feature of the signal and a coefficient associated with the stimulation from a result of processing obtained in b).
(18A) The program of item 18, further comprising a characteristic of any one or more of items 1 to 15.

(19) A program for making a computer perform a method of analyzing an object, the method comprising:

a) obtaining a signal in response to stimulation from the object;
b) applying cross-correlation processing to the signal using a part or all of the signal;
c) calculating a feature of the signal and a coefficient associated with the stimulation from a result of processing obtained in b); and
d) analyzing a reaction to the stimulation of the object using the feature and the coefficient.
(19A) The program of item 19, further comprising a characteristic of any one or more of items 1 to 15.

(20) A recording medium for storing a program for making a computer perform a method of processing a signal of an object in response to stimulation, the method comprising:

a) obtaining a signal in response to stimulation from the object;
b) applying cross-correlation processing to the signal using a part or all of the signal; and
c) calculating a feature of the signal and a coefficient associated with the stimulation from a result of processing obtained in b).
(20A) The recording medium of item 20, further comprising a characteristic of any one or more of items 1 to 15.

(21) A recording medium for storing a program for making a computer perform a method of analyzing an object, the method comprising:

a) obtaining a signal in response to stimulation from the object;
b) applying cross-correlation processing to the signal using a part or all of the signal;
c) calculating a feature of the signal and a coefficient associated with the stimulation from a result of processing obtained in b); and
d) analyzing a reaction to the stimulation of the object using the feature and the coefficient.
(21A) The recording medium of item 21, further comprising a characteristic of any one or more of items 1 to 15.

The present invention is intended so that one or more of the aforementioned characteristics can be provided not only as the explicitly disclosed combinations, but also as other combinations thereof. Additional embodiments and advantages of the invention are recognized by those skilled in the art by reading and understanding the following detailed description as needed.

[Advantageous Effects of Invention]

The present invention can accurately monitor the change in subjective pain simultaneously (in real-time) without separate measurement of a control. The present invention can also administer more detailed therapy or surgery matching the subjective evaluation based on the accurately recorded change in pain, so that the present invention is useful in the medicine related industries.

[Brief Description of Drawings]

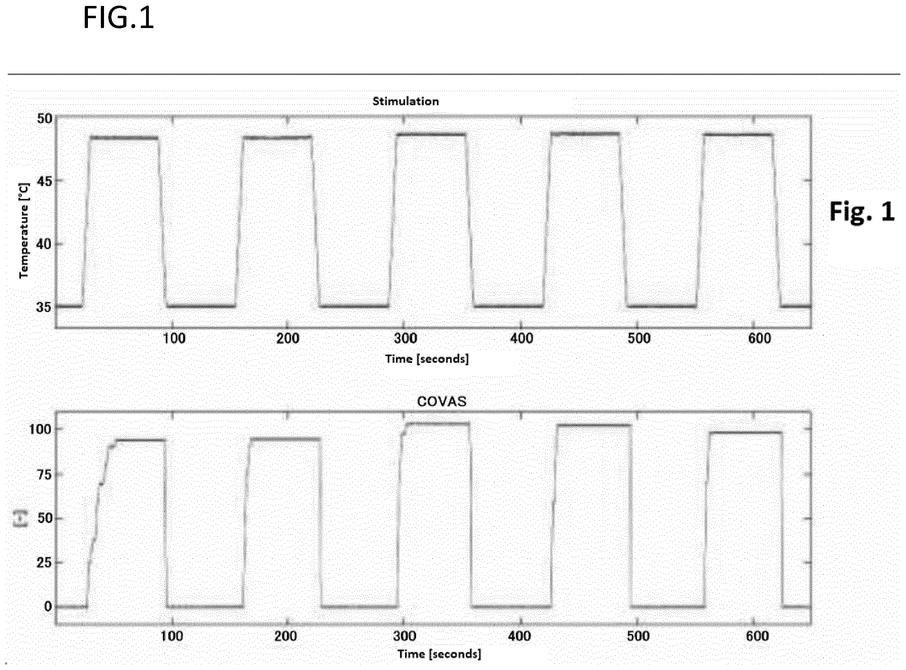

[Figure 1] Figure **1** is a diagram showing a comparison between thermal stimulation pattern and VAS.

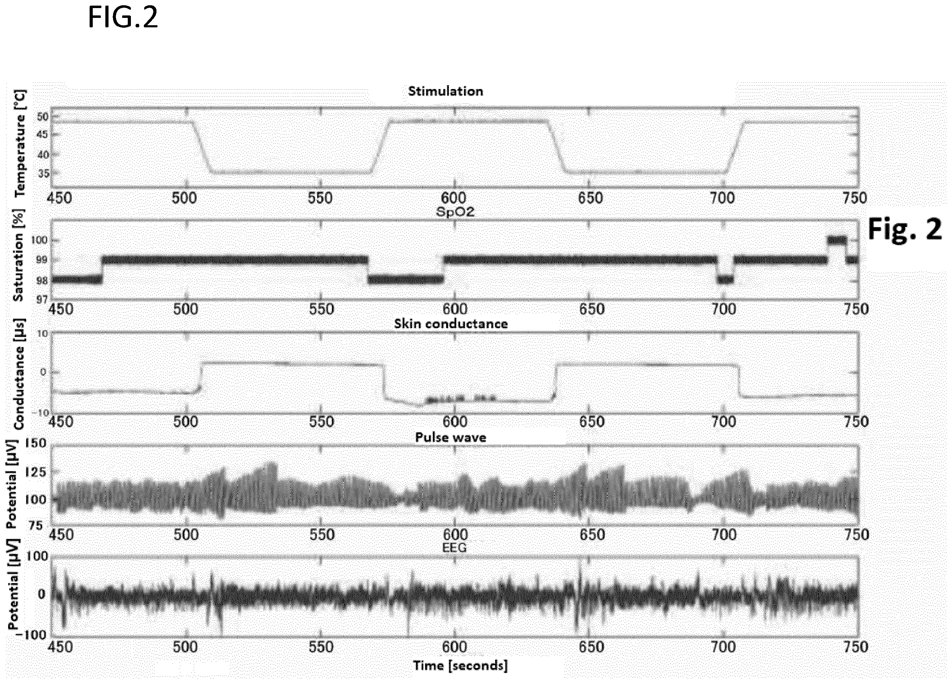

[Figure 2] Figure **2** shows all the biological signals collected in Example 1. From the top, stimulation, $SpO_2$, skin conductance, pulse wave, and EEG are shown, respectively.

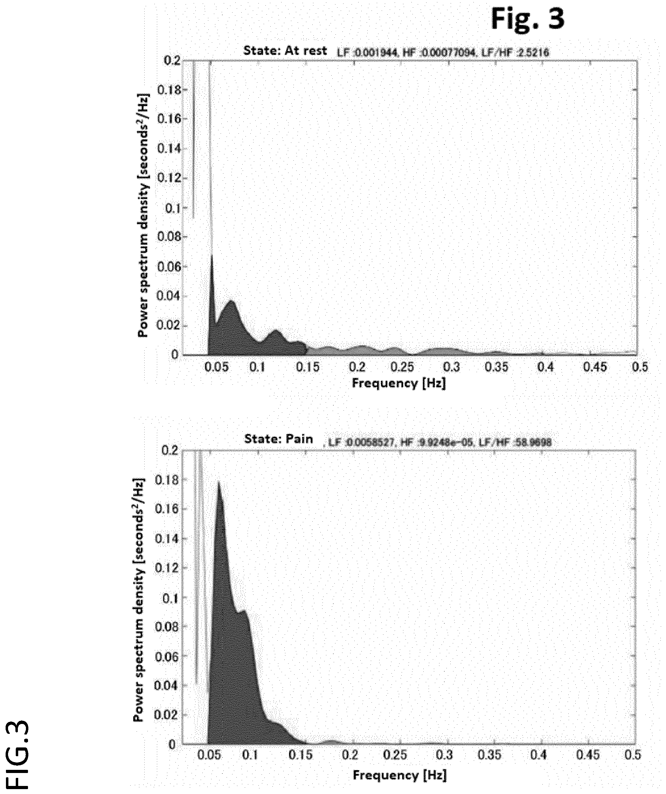

[Figure 3] Figure **3** shows the LH/HF and R-R interval frequency accompanying stimulation in the top panel, and LH/HF and R-R interval frequency with no pain in the bottom panel.

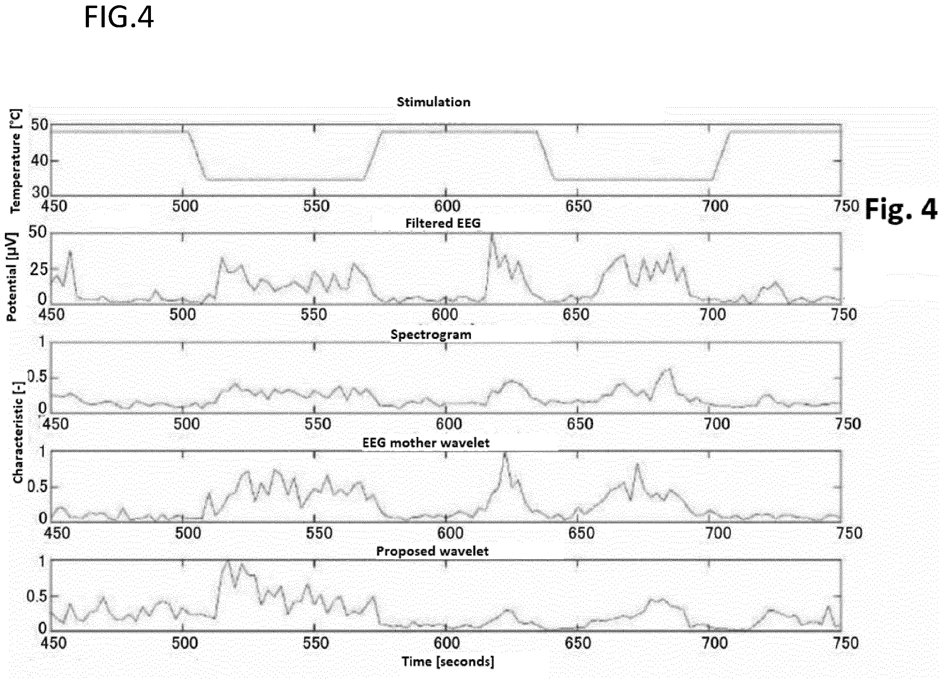

[Figure 4] Figure **4** shows features obtained by EEG.

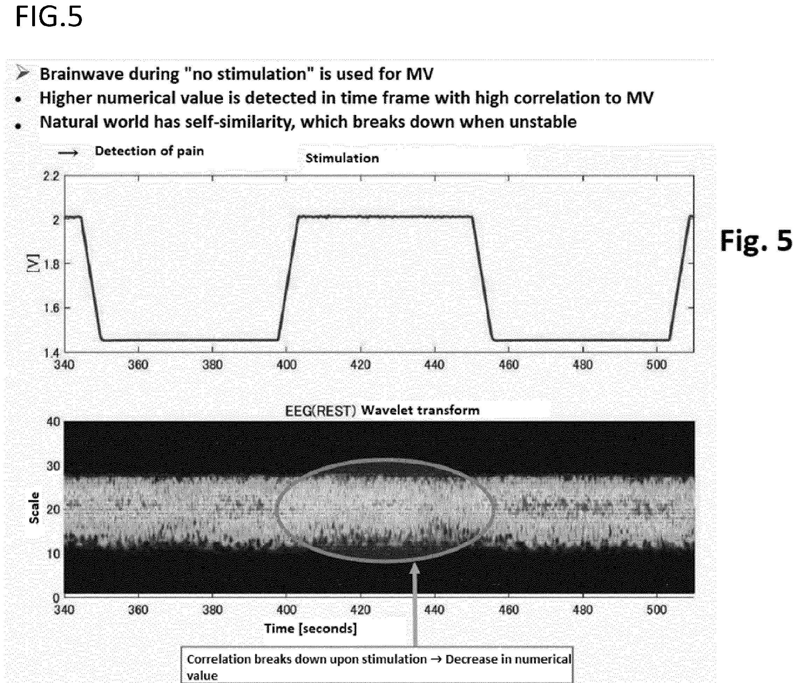

[Figure 5] Figure 5 shows preliminary experimental results. This is a result when using a brainwave upon "no stimulation" for the mother wavelet (MW). A higher numerical value is detected in a time frame with high correlation with MW. It was found that the natural world has self-similarity, which breaks down when unstable. The present invention applies this to detection of pain.

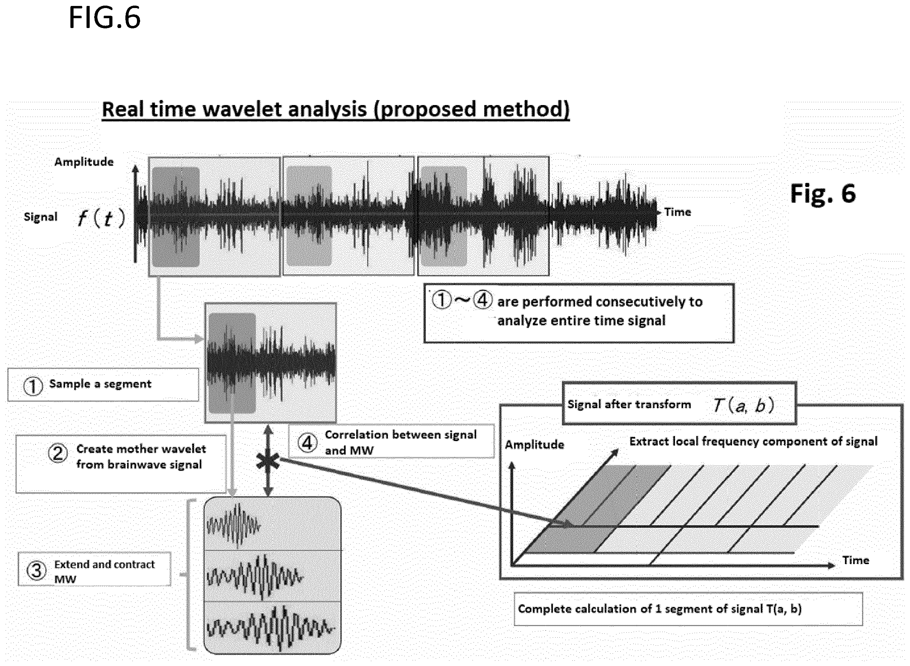

[Figure 6] Figure **6** is a schematic diagram of the real-time wavelet analysis of the invention.

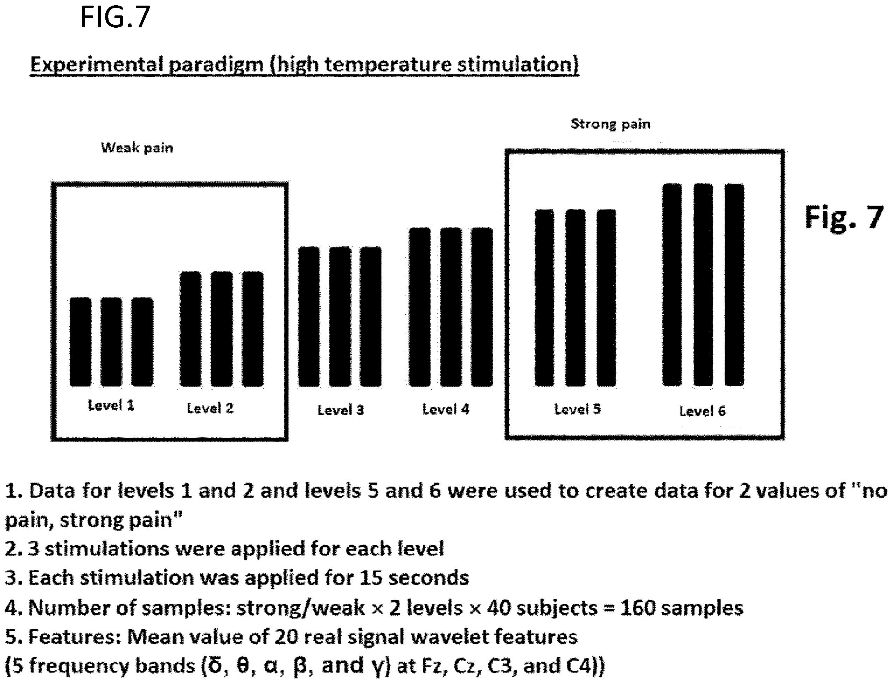

[Figure 7] Figure **7** shows an experimental paradigm using high temperature stimulation, which includes six levels of pain levels. Each level includes three stimulations. Each stimulation lasted 15 seconds. Differentiation analysis of pain levels included levels 1 and 2 as "weak pain" conditions, and levels 5 and 6 as "strong pain" conditions. There were 40 participants, and samples used for differentiation were 160 in total. As the features, 20 features of four electrodes at Fz, Cz, C3, and C4 and five frequency bands ($\delta$, $\theta$, $\alpha$, $\beta$, $\gamma$) were used.

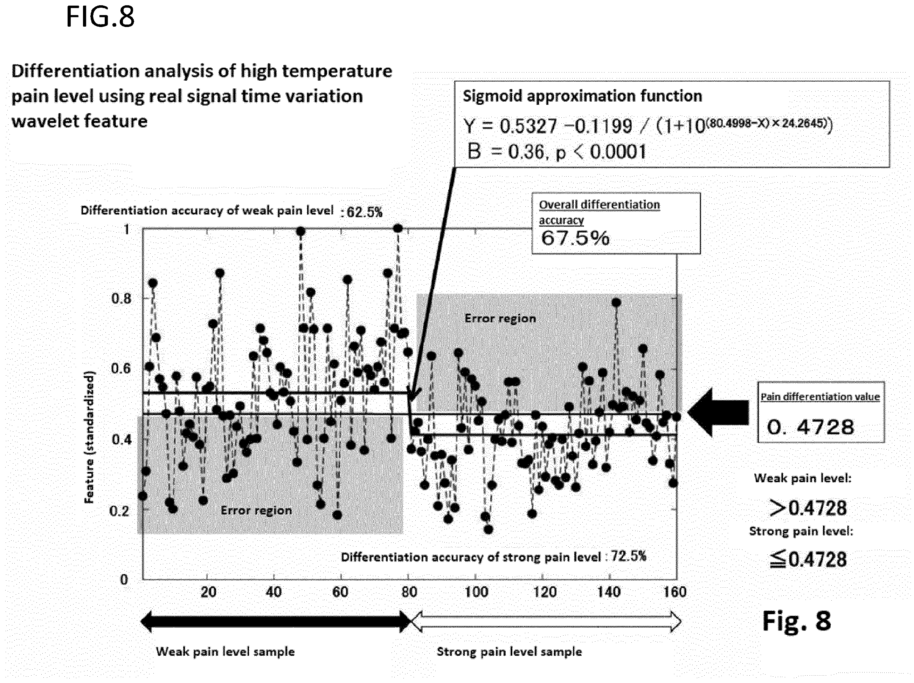

[Figure 8] Figure **8** shows results of differentiation analysis of two levels of high temperature pain (160 samples) using a real signal wavelet feature and sigmoid function. The differentiation value corresponding to an inflection point of a sigmoid approximation function is "0.4728", and an overall differentiation accuracy of "67.5%" was materialized.

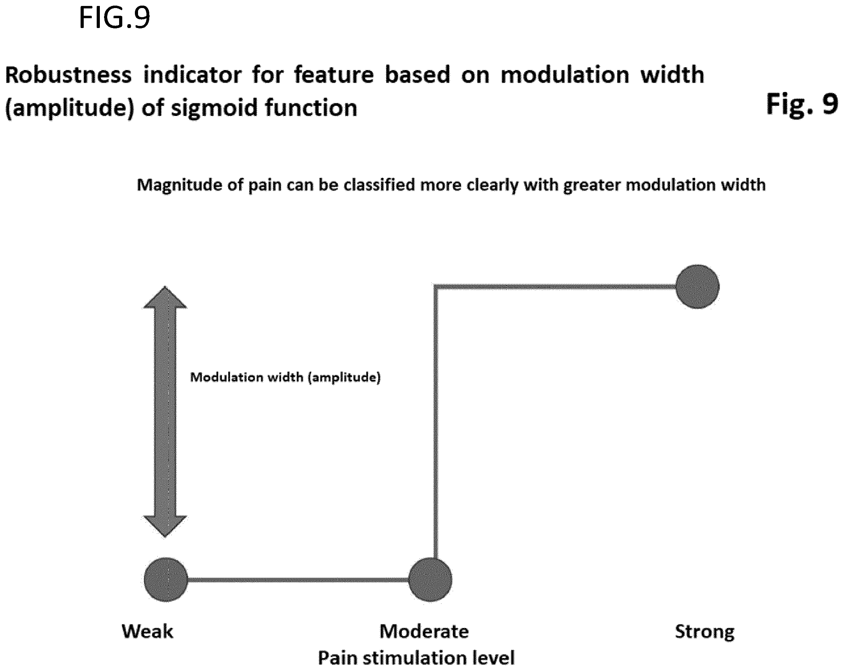

[Figure 9] Figure **9** is a robustness indicator associated with differentiation accuracy of a feature based on the modulation width of a sigmoid function. A greater modulation width between the minimum approximation value and maximum approximation value results in higher differentiation accuracy, with a greater difference in features associated with magnitude of pain.

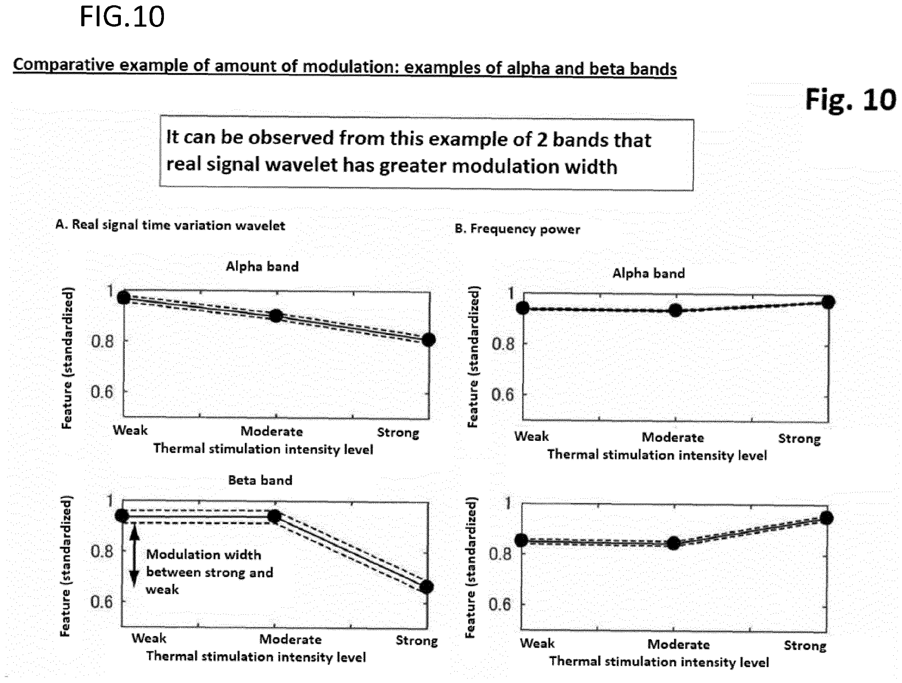

[Figure 10] Figure **10** shows a change in features in the $\alpha$ band and $\beta$ band involving changes in the pain level. A real signal wavelet feature has a greater modulation width involving a change from weak pain level to strong pain level compared to a frequency power feature.

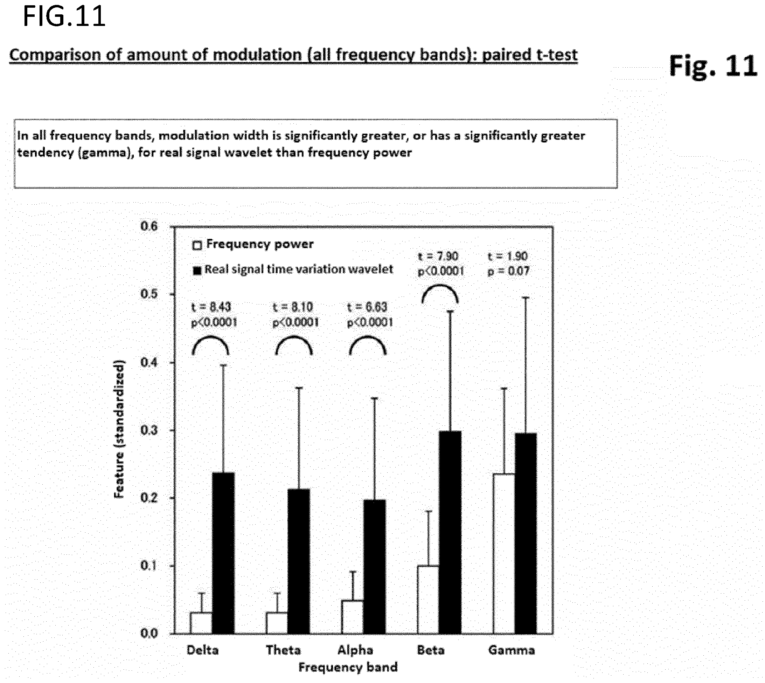

[Figure 11] Figure **11** shows results of a comparison test on the modulation widths of real signal wavelet and frequency power features. The real signal wavelet tends to have a significantly greater modulation width in all frequency bands.

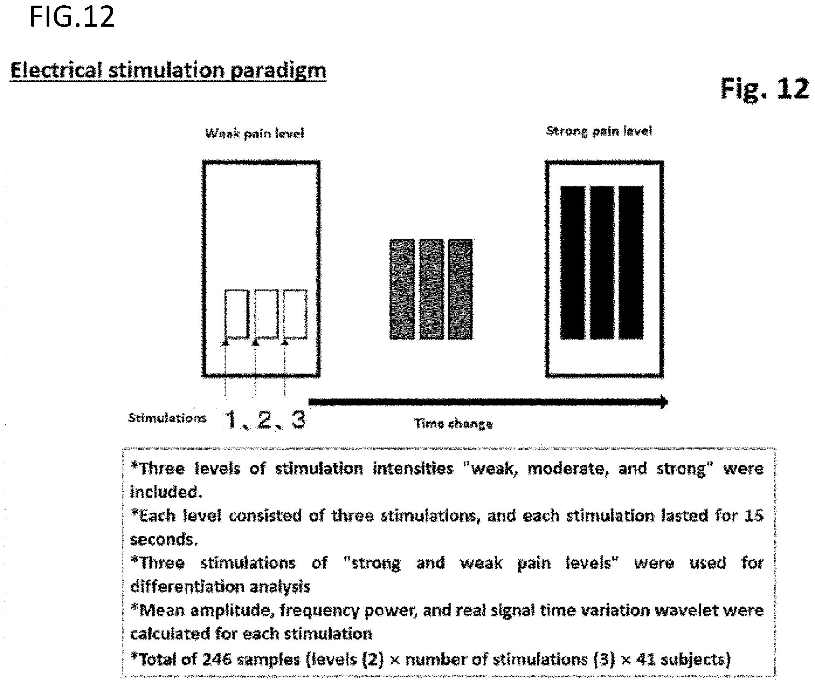

[Figure 12] Figure **12** shows a method of an electrical stimulation paradigm (three levels). The experimental paradigm included three intensity levels (weak $\rightarrow$ strong), and each level included three stimulations. Each stimulation application time was 15 seconds. Three stimulations of "weak and strong pain levels" were independently used for differentiation analysis. A total of 246 samples (strong/weak level (2) $\times$ number of stimulations (3) $\times$ 41 subjects) were differentiated. The mean amplitude, frequency power, and real signal wavelet feature were calculated for each stimulation.

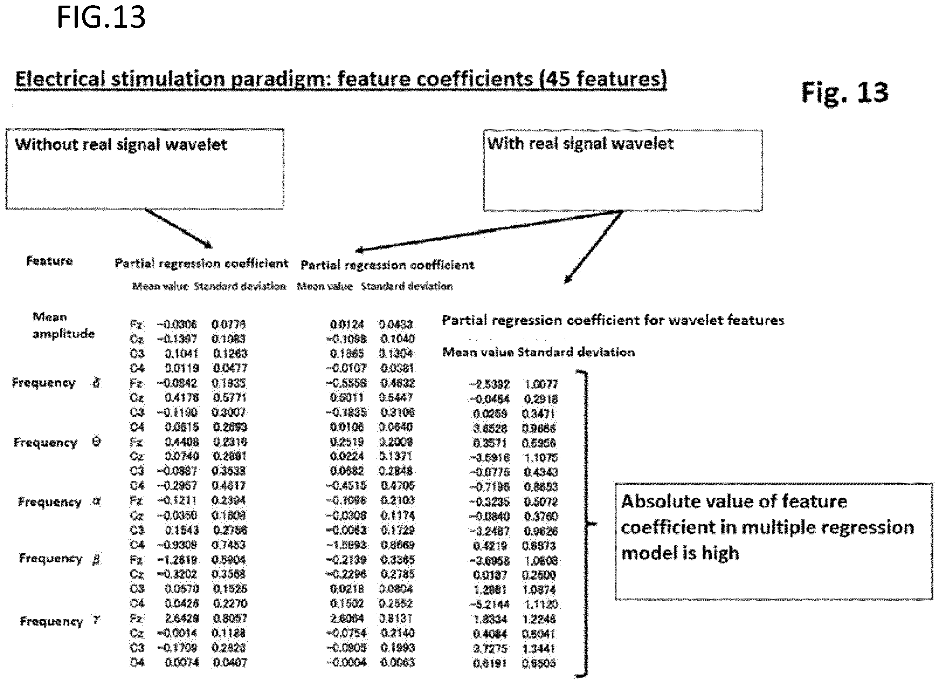

[Figure 13] Figure **13** shows a result for feature coefficients (45 features) in a differentiation model of electrical stimulation pain levels. The left side shows differentiation analysis with only a mean amplitude and frequency power without a real signal wavelet, and the right side shows a case where a real signal wavelet feature was inputted. It can be understood that the absolute value of a feature coefficient is higher and contribution to a model is high when a wavelet feature is added to analysis.

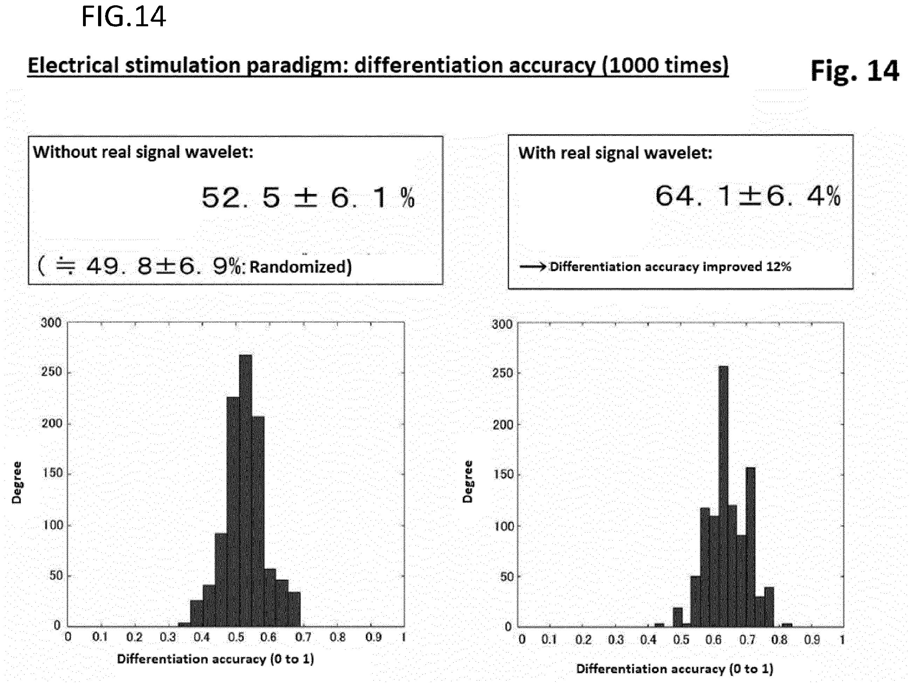

[Figure 14] Figure **14** shows results of 1000 differentiation accuracy tests for two levels (weak and strong) of electrical stimulation. The left side shows a case where a real signal wavelet is not added, and the right side shows a case where a real signal wavelet is added. The differentiation accuracy improved about 10% just by adding a wavelet, exhibiting a differentiation accuracy of about 65%.

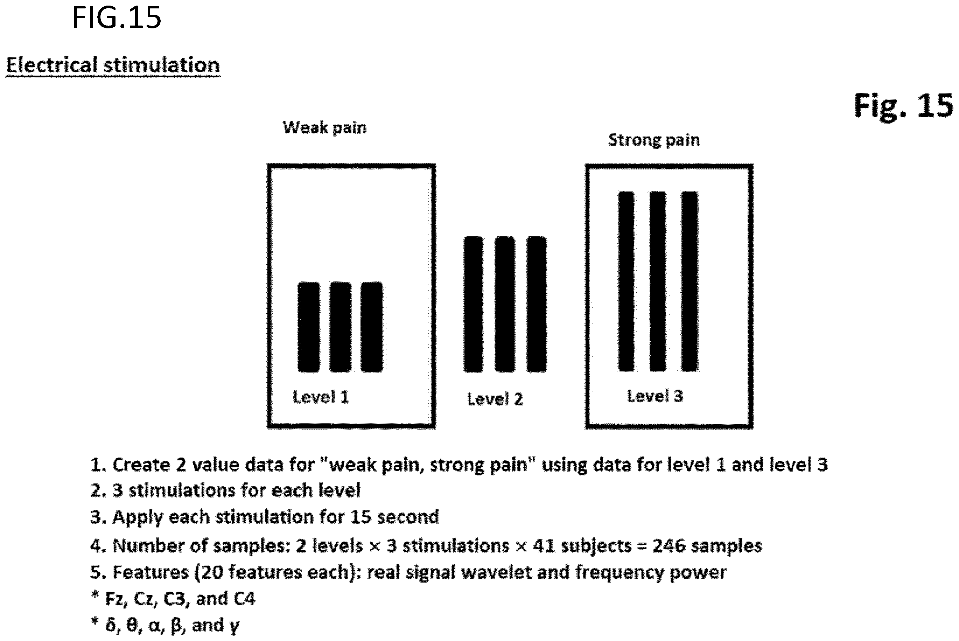

[Figure 15] Figure **15** shows an electrical stimulation experimental paradigm used in pain level differentiation by machine learning, including three intensity levels. Each level included three stimulations. The stimulation application time was 15 seconds. Three stimulations of "weak and strong pain levels" were independently used for differentiation analysis. A total of 246 samples were differentiated. Differentiation analysis was performed using real signal wavelet features and qualitatively/quantitatively corresponding frequency power for comparison thereof.

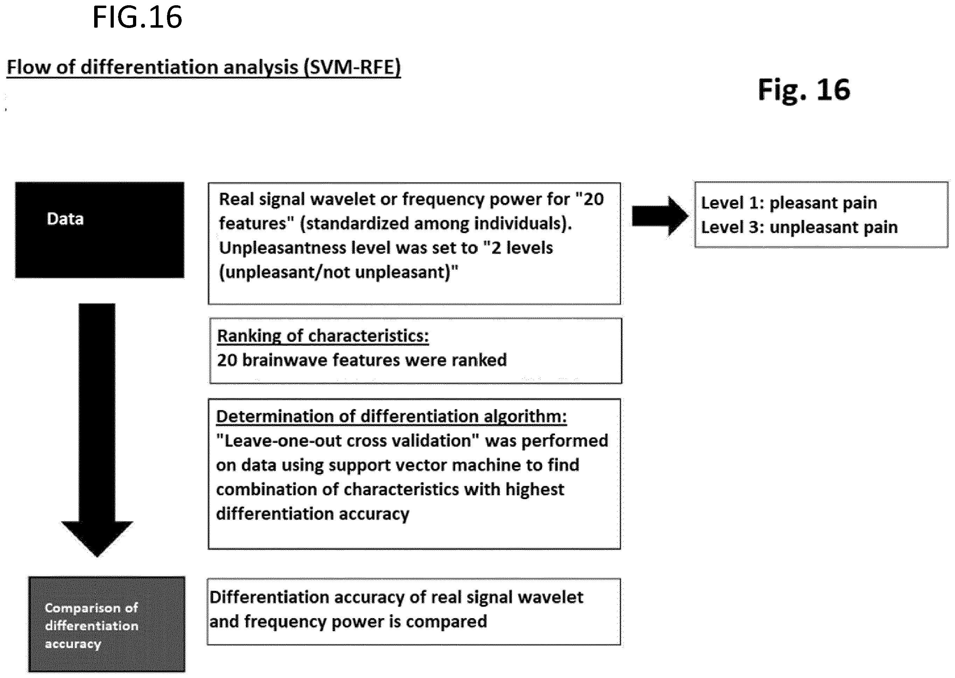

[Figure 16] Figure **16** shows the flow of differentiation analysis. Supervised machine learning, i.e., Support Vector Machine Recursive Feature Elimination (SVM RFE), was used for the differentiation analysis. A differentiation model for 246 samples related to two levels (strong/weak) of pain was trained using 20 real signal wavelets to investigate the differentiation accuracy. First, the contribution of 20 features in a model was recursively aggregated to find the ranking of all 20 features. Features were increased one at a time from the top ranking features to perform leave-one-out cross validation of 246 samples.

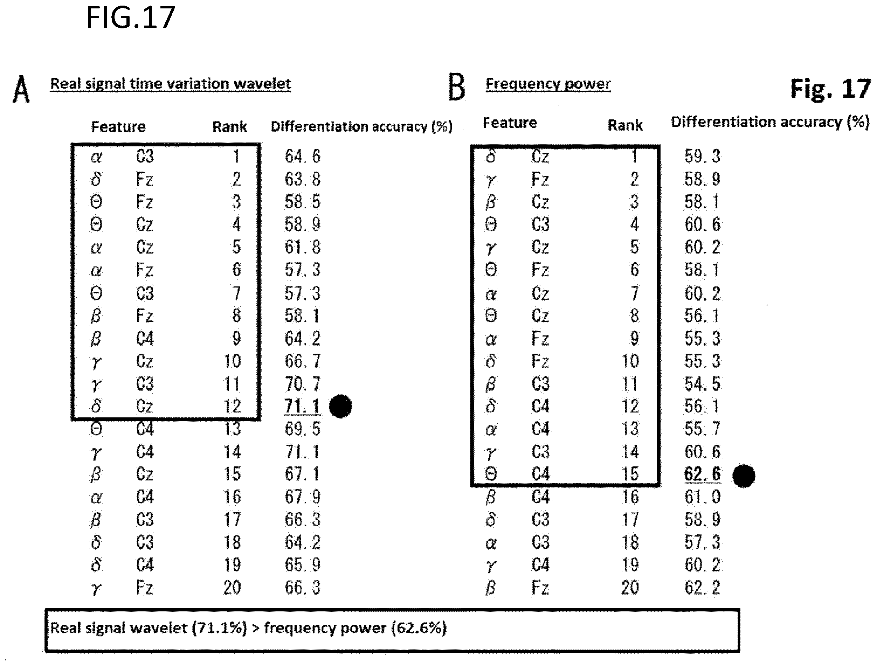

[Figure 17] Figure **17** shows a comparative result of differentiation accuracy in SVM using real signal wavelets and frequency power. The highest differentiation accuracy when using a real signal wavelet was "71.1%", involving 12 features. Meanwhile for frequency power, the highest differentiation accuracy was "62.6%" which was about a 10% decrease in differentiation accuracy. Three more features are used, demonstrating that this is an uneconomical model with low differentiation accuracy.

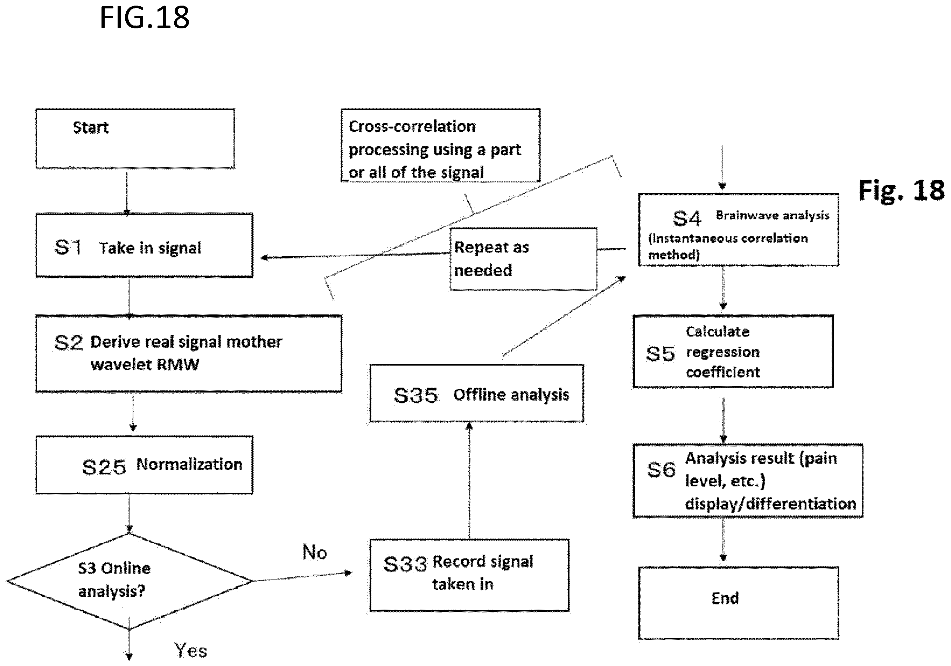

[Figure 18] Figure **18** is a flow chart showing the method of the invention.

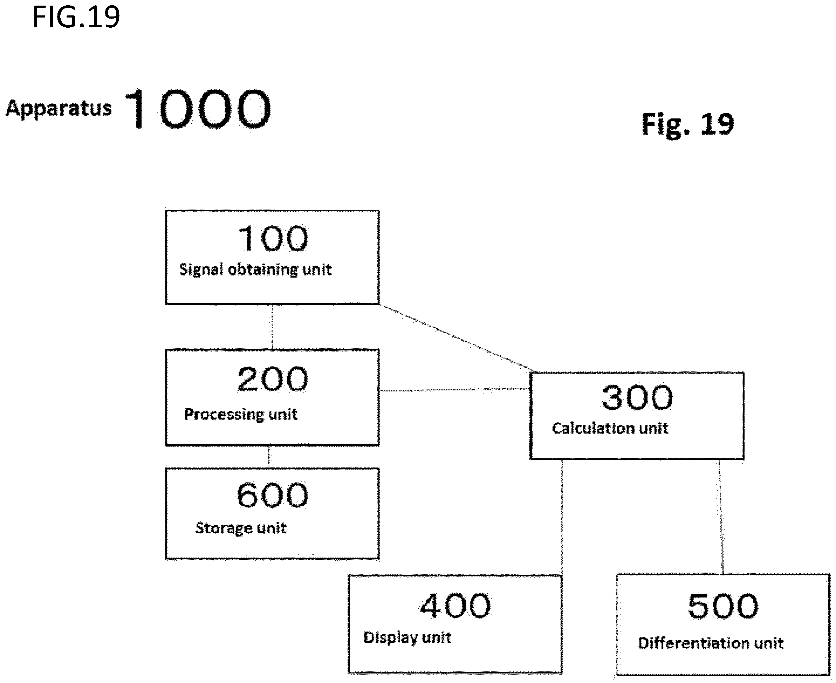

[Figure 19] Figure **19** is a block diagram showing the configuration of the apparatus of the invention.

[Description of Embodiments]

The present invention is explained hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

(Definition)

The terms and the general technologies used herein are first explained.

As used herein, "object" refers to an entity subjected to analysis of signals of the invention. Any entity that emits a signal (e.g., organism when analyzing pain, pleasantness/unpleasantness, emotion, or the like in the organism (e.g., brainwave thereof and the like), machinery when analyzing an engine, breakdown in harmonic vibration, or the like, earth or ground in case of earthquake, and the like) is encompassed in the scope of object. For organisms and humans, an object is also referred to as a patient or subject. If the purpose is to analyze pain, an object refers to any organism or animal targeted by the technology herein such as pain measurement and brainwave measurement. An object is preferably, but is not limited to, humans. As used herein, an object may be referred to as an "object being estimated" when estimating pain, but this has the same meaning as object or the like.

As used herein, "signal" is used in the common meaning in the art, referring to the quantity or condition used for communicating or expressing information. Signals take various forms such as voltage, current, amplitude of various waves, position of instrument pointer, open/close state of a switch, and chemical composition. Typically, a signal has a property of a wave and is also referred to as a signal wave, and has the ability to communicate information, report, effect, or the like. When an object is the brain, a signal is also referred to as a brainwave (signal) or the like.

As used herein, "brainwave" has the meaning that is commonly used in the art and refers to a current generated by a difference in potential due to neurological activity of the brain when a pair of electrodes is placed on the scalp. Brainwave encompasses electroencephalogram (EEG), which is obtained from deriving and recording temporal changes in the current. A wave with an amplitude of about 50 $\mu$V and a frequency of approximately 10 Hz is considered the primary component at rest. This is referred to an $\alpha$ wave. During neurological activity, $\alpha$ waves are suppressed and a fast wave with a small amplitude of 17 to 30 Hz appears, which is referred to as a $\beta$ wave. It is understood that during a period of shallow sleep, $\alpha$ waves gradually decrease and $\theta$ waves of 4 to 8 Hz appear. It is understood that during a deep sleep, $\delta$ waves of 1 to 4 Hz appear. These brainwaves can be described by a specific amplitude and frequency.

As used herein, "brainwave data" is any data related to brainwaves (also referred to as "amount of brain activity", "brain feature", or the like), such as amplitude data (EEG amplitude, frequency property, or the like). "Analysis data" from analyzing such brainwave data can be used in the same manner as brainwave data, so that they can be collectively referred to as "brainwave data or analysis data thereof" herein. Examples of analysis data include mean amplitude or peak amplitude of brainwave data (e.g., Fz, Cz, C3, C4), frequency power (e.g., $Fz(\delta)$, $Fz(\theta)$, $Fz(\alpha)$, $Fz(\beta)$, $Fz(\gamma)$, $Cz(\delta)$, $Cz(\theta)$, $Cz(\alpha)$, $Cz(\beta)$, $Cz(\gamma)$, $C3(\delta)$, $C3(\theta)$, $C3(\alpha)$, $C3(\beta)$, $C3(\gamma)$, $C4(\delta)$, $C4(\theta)$, $C4(\alpha)$, $C4(\beta)$, $C4(\gamma)$)) and the like. Of course, this does not exclude other data commonly used as brainwave data or analysis data thereof. The real signal wavelet in the present invention can also be considered brainwave data derived based on the primary brainwave data described above, so that the real signal wavelet can be considered as "secondary brainwave data" or "derived brainwave data".

As used herein, "amplitude data" is one type of wave data that exhibits an important function in a brainwave or the like. Representative examples thereof include data for amplitudes of brainwaves. This is also referred to as simply "amplitude" or "EEG amplitude" for brainwaves. Since such amplitude data is an indicator of brain activity, such data can also be referred to as "brain activity data", "amount of brain activity", or the like. For brainwaves, amplitude data can be obtained by measuring electrical signals of a brainwave and is indicated by potential (can be indicated by μV or the like). Amplitude data that can be used include, but are not limited to, mean amplitude.

As used herein, "frequency power" expresses frequency components of a waveform as energy and is also referred to as power spectrum. Frequency power can be calculated by extracting and calculating frequency components of a signal embedded in a signal containing noise within a time region by utilizing fast Fourier transform (FFT) (algorithm for calculating discrete Fourier transform (DFT) on a computer at high speeds). FFT on a signal can, for example, use the function periodgram in MATLAB to normalize the output thereof and calculate the power spectrum density (PSD) or power spectrum, which is the source of measurement of power. PSD indicates how power of a time signal is distributed with respect to frequencies. The unit thereof is watt/Hz. Each point in PSD is integrated over the range of frequencies where the point is defined (i.e., over the resolution bandwidth of PSD) to calculate the power spectrum. The unit of a power spectrum is watt. The value of power can be read directly from power spectrum without integration over the range of frequencies. PSD and power spectrum are both real numbers, so that no phase information is included. In this manner, frequency power can be calculated with a standard function in MATLAB. Such frequency power can be targeted in the technology of the invention.

"Pain" refers to a sensation that is generated as stimulation, generally upon intense injury such as damage/inflammation to a body part. In humans, pain is encompassed in common sensations as a sensation accompanying strong unpleasant feeling. In addition, cutaneous pain and the like also has an aspect as an external receptor to a certain degree, which plays a role in determining the quality such as hardness, sharpness, hotness (thermal pain), coldness (cold pain), or spiciness of an external object in cooperation with other skin sensation or taste. The sensation of pain of humans can occur at almost any part of the body (e.g., pleura, peritoneum, internal organs (visceral pain, excluding the brain), teeth, eyes, ears, and the like) other than the skin and mucous membrane, which can all be sensed as a brainwave or a change thereof in the brain. Additionally, internal sensation of pain represented by visceral pain is also encompassed by sensation of pain. The aforementioned sensation of pain is referred to as somatic pain relative to visceral pain. In addition to somatic pain and visceral pain, sensation of pain called "referred pain", which is a phenomenon where pain is perceived at a surface of a site that is different from a site that is actually damaged, is also reported. The present invention provides a methodology of objectively measuring or differentiating subjective pain levels without performing a control experiment involving pain or the like in real-time.

For sensation of pain, there are individual differences in sensitivity (pain threshold), as well as qualitative difference due to a difference in the receptor site or how a pain stimulation occurs. Sensation of pain is classified into dull pain, sharp pain, and the like, but sensation of pain of any type can be measured, estimated, and classified in this disclosure. The disclosure is also compatible with fast sensation of pain (A sensation of pain), slow sensation of pain (B sensation of pain), (fast) topical pain, and (slow) diffuse pain. The present invention is also compatible with abnormality in sensation of pain such as hyperalgesia. Two nerve fibers, i.e., "Aδ fiber" and "C fiber", are known as peripheral nerves that transmit pain. For example, when a hand is hit, the initial pain is transmitted as sharp pain from a clear origin (primary pain: sharp pain) by conduction through the Aδ fiber. Pain is then conducted through the C fiber to feel throbbing pain (secondary pain; dull pain) with an unclear origin. Pain is classified into "acute pain" lasting 4 to 6 weeks or less and "chronic pain" lasting 4 to 6 weeks or more. Pain is an important vital sign along with pulse, body temperature, blood pressure, and breathing, but is difficult to express as objective data. Representative pain scales VAS (Visual Analogue Scale) and faces pain rating scale are subjective evaluation methods that cannot objectively evaluate or compare pain between subjects. These methods presume applying pain to a subject, which are highly invasive and have low compliance. In view of the above, the inventors developed a methodology that can analyze brainwaves simultaneously (in real-time) without pre-processing as an indicator for objective evaluation of pain, leading to the ability to differentiate and classify any type of pain. Instantaneous stimulation and persistent stimulation can also be detected.

As used herein, "subjective pain sensation level" refers to the level of sensation of pain of an object, and can be expressed by conventional technology such as computerized visual analog scale (COVAS) or other known technologies such as Support Team Assessment Schedule (STAS-J), Numerical Rating Scale (NRS), Faces Pain Scale (FPS), Abbey pain scale (Abbey), Checklist of Nonverbal Pain Indicators (CNPI), Non-communicative Patient's Pain Assessment Instrument (NOPPAIN), Doloplus 2, or the like. The technology of the invention provides a technology that can replace these subjective pain sensation levels.

One of the important points of the present invention is the ability to distinguish whether pain is pain "requiring therapy" nearly in real-time. Therefore, it is important that "pain" can be clearly categorized based on the concept of "therapy". Since pain can be precisely differentiated, the present invention is a technology that is capable of "qualitative" classification of pain such as "pleasant/unpleasant" or "unbearable". The real signal wavelet feature in the present invention improves the accuracy to differentiate qualitative and quantitative difference in pain and improves the detection capability by using a differentiation model.

As used herein, "stimulation" includes any stimulation that can cause sensation of pain. Examples thereof include electrical stimulation, cold stimulation, thermal stimulation, physical stimulation, chemical stimulation, and the like. In the present invention, stimulation can be any stimulation. Evaluation of stimulation can be matched with subjective pain sensation levels using, for example, conventional technology such as computerized visual analog scale (COVAS) or other known technologies such as Support Team Assessment Schedule (STAS-J), Numerical Rating Scale (NRS), Faces Pain Scale (FPS), Abbey pain scale (Abbey), Checklist of Nonverbal Pain Indicators (CNPI), Non-communicative Patient's Pain Assessment Instrument (NOPPAIN), Doloplus 2, or the like. Examples of values that can be employed as stimulation intensity include nociceptive threshold (threshold for generating neurological impulses in nociceptive fiber), pain detection threshold (intensity of nociceptive stimulation that can be sensed as pain by humans), pain tolerance threshold (strongest stimulation intensity among nociceptive stimulation that is experimentally tolerable by humans), and the like.

As used herein, "cross-correlation processing" refers to derivation of a relationship of cross-correlation with some type of an object. Cross-correlation processing refers to performing correlation processing on two signal waveforms to find the similarity between the two waveforms. This includes cases where the target of cross-correlation processing is itself. In such a case, this is referred to as autocorrelation processing.

As used herein, "autocorrelation processing" refers to correlation processing on one signal waveform to find the degree of similarity of a part of the signal to other parts.

As used herein, "self-similar(ity)" refers to the similarity of the whole to a portion in some context. A representative figure includes fractals.

As used herein, "feature" is any characteristic of an object that is quantified. For the brain, examples thereof include brainwave data and analysis data thereof and the like. Examples thereof include mean amplitude and peak amplitude (e.g., Fz, Cz, C3, C4), frequency power (e.g., $Fz(\delta)$, $Fz(\theta)$, $Fz(\alpha)$, $Fz(\beta)$, $Fz(\gamma)$, $Cz(\delta)$, $Cz(\theta)$, $Cz(\alpha)$, $Cz(\beta)$, $Cz(\gamma)$, $C3(\delta)$, $C3(\theta)$, $C3(\alpha)$, $C3(\beta)$, $C3(\gamma)$, $C4(\delta)$, $C4(\theta)$, $C4(\alpha)$, $C4(\beta)$, $C4(\gamma)$) and the like of brainwave data. The real signal wavelet feature in the present invention can also be considered as a "secondary feature" or "derived feature" derived from a primary feature described above, which is a specific amplified brain signal property. Such secondary features and derived features can also be used in the present invention.

As used herein, "coefficient" refers to a coefficient (e.g., regression coefficient) associated with each feature. A coefficient indicates the degree of association of each feature involved in the analysis of an object. Analysis can be performed by "fitting" to a suitable function. For example, a technique of fitting a measured value or a curve obtained therefrom to approximate a function is referred to as "fitting", which can be performed based on any approach. For example, a known sigmoid fitting function can be used. Examples of such fitting include least square fitting, nonlinear regression fitting (MATLAB's nlinfit function or the like), and the like.

As used herein, "coefficient associated with stimulation" refers to a coefficient associated with stimulation causing pain stimulation (e.g., cold stimulation, physical stimulation, or the like) and stimulation level such as the intensity or degree of unpleasantness thereof, or a corresponding feature.

After fitting, a regression coefficient can be calculated for the approximated curve to determine whether the curve can be used or preferable in the present invention. For a regression coefficient, a regression equation model is effective. The adjusted coefficient of determination ($R^2$) is desirable with a numerical value closer to "1" such as 0.5 or greater, 0.6 or greater, 0.7 or greater, 0.8 or greater, 0.85 or greater, 0.9 or greater, or the like. A higher numerical value has higher confidence. The accuracy of fitting can be studied by using a specific threshold value to categorize and compare an estimated value and an actual measurement value (this is referred to as differentiation accuracy in the analysis of the invention). In such a case, all features can be averaged and consolidated to create a fitting function used in differentiation. Meanwhile, features can be compared and examined with respect to each other by performing fitting for individual features to indicate the degree of approximation with a coefficient and calculate differentiation accuracy of pain levels.

As used herein, "wavelet (transform, analysis, method)" is a frequency analysis methodology for expanding/contracting, translating, and adding together small waves (wavelets) to express a waveform of a given input. A mother wavelet (analyzing wavelet) is generally used as the basis function. While a basis function with several cycles is used in analysis for Fourier transform, wavelets have one cycle. As a result, multiresolution results can be obtained so that useful information such as a discontinuous point can be retrieved. The present invention also found that wavelet analysis can remove noise and extract meaning signals, and extract information on pain contained in a brainwave or the like. Explanation herein is described in detail in the following sections.

As used herein, "real signal wavelet (analysis, transform)" refers to a wavelet (analysis, transform) used in an actual signal as a basis function.

As used herein, "time variation wavelet (analysis, transform)" refers to a wavelet (analysis, transform) created differently for each time frame. Analysis using such a wavelet is referred to as time variation wavelet analysis.

As used herein, "classification" of pain can be performed from various points of view. Representative examples include classification by whether pain is "painful" or "not painful" for the object being estimated, but a methodology of classification for pain felt by whether the pain is strong pain or weak pain, or "bearable" pain or "unbearable" pain can be additionally envisioned. Other examples include a methodology of classification between "painful and unpleasant" and "painful but pleasant". The present disclosure can be used to chronologically determine/estimate whether an object feels unbearable strong pain or weak pain by observing a monotonous increase or monotonous decrease.

(Preferred embodiments)

The preferred embodiments of the present invention are described hereinafter. It is understood that the embodiments provided hereinafter are provided for better understanding of the present invention, so that the scope of the invention should not be limited by the following descriptions. Thus, it is apparent that those skilled in the art can refer to the descriptions herein to make appropriate modifications within the scope of the invention. It is also understood that the following embodiments of the invention can be used individually or as a combination.

Each of the embodiments described below provides a comprehensive or specific example. The numerical values, shapes, materials, constituent elements, positions of arrangement and connection forms of the constituent elements, steps, order of steps, and the like in the following embodiments are one example, which is not intended to limit the Claims. Further, the constituent elements in the following embodiments that are not recited in the independent claims showing the most superordinate concept are described as an optional constituent element.

(Simultaneous cross-correlation signal processing)

In one aspect, the present invention provides a method of processing a signal of an object in response to stimulation, comprising: A) obtaining a signal in response to stimulation from the object; B) applying cross-correlation processing to the signal using a part or all of the signal; and C) calculating a feature of the signal and a coefficient associated with the stimulation from a result of processing obtained in b). Cross-correlation processing includes cross-correlation processing that is autocorrelation processing and non-autocorrelation processing. For cross-correlation processing that is non-autocorrelation processing, analysis can be performed while creating a basis using channels with different brainwave signals. Specific conceivable examples thereof include correlation processing which analyze the frontal lobe with a basis from the occipital portion and the like.

In one embodiment, the cross-correlation processing used in the present invention comprises autocorrelation processing. In one embodiment, the correlation processing of the invention (including cross-correlation processing and autocorrelation processing) comprises finding self-similarity for each time.

In one embodiment, the signal targeted by the present invention does not have self-similarity. Examples thereof include brainwave data when detecting pain. In addition, an organism when analyzing pleasantness/unpleasantness, emotion, or the like (e.g., brainwave thereof and the like), machinery when analyzing an engine, breakdown in harmonic vibration, or the like, earth or ground in case of earthquake, and the like can be targeted.

In one embodiment, the signal is a biological signal. Biological signals often indicate an abnormal condition when self-similarity is lost. It is an important effect that these can be instantaneously determined in real-time. Preferably, a biological signal is a brain signal.

In one embodiment, correlation processing (including cross-correlation processing and autocorrelation processing) in the present invention can be performed using real signal wavelet transform. Real signal wavelet transform is a methodology using a real signal as a basis function among wavelet analysis. This is in contrast to a methodology using an existing function such as Garbor function. Wavelet transform includes continuous and discrete wavelet transform, both of which can be used in the present invention. Preferably, continuous wavelet transform is used. Continuous wavelet transform is relatively easily handled in a computer, and is generally capable of more detailed analysis than discrete wavelet transform. Meanwhile, inverse transformation can be performed in discrete wavelet transform. Information that has been transformed once can be processed and inversely transformed for application in noise removal or the like.

More specifically, a wavelet coefficient in continuous wavelet transform is given by

[Numeral 1]

$$T(a,b) = \frac{1}{\sqrt{a}} \int_{-\infty}^{\infty} x(t)\psi^*\left(-\frac{b-t}{a}\right) dt$$

wherein x(t) represents a time domain signal of a signal targeted for analysis (e.g., EEG), and a and b are expansion and translation parameters, respectively. Parameter a correlates with frequency, and b correlates with time. * indicates a complex conjugate. Any wavelet can be used as the mother wavelet $\psi$(t), but Morelet wavelet, Mexican hat, Haar, Meyer-Lemarie, Paul, DOG (Derivative of Gaussian), or the like can be used. A Morlet wavelet is given by

[Numeral 2]

$$\psi(t) = e^{-t^2/2} \cos 5t$$

.

Parameters a and b are associated with translation and expansion of the mother wavelet, respectively.

One of the characteristics of the present invention is in simultaneously performing wavelet analysis without pre-processing. This is also referred to as "simultaneous wavelet analysis". The conventionally used instantaneous correlation method performs the analysis itself instantaneously, but presumes obtaining control or reference data in advance. Meanwhile, one of the characteristics of the present invention is that this is not required, where imported data is simultaneously subjected to wavelet analysis and correlation of the instantaneous correlation method or the like for analysis. Such a methodology can be used under a circumstance where it is not desirable to, or is challenging to set conditions in advance, such as measurement of pain. The methodology can be applied to, as such circumstances, pain, as well as analysis of pleasantness/unpleasantness, emotion, or the like in an organism, analysis of an engine, breakdown in harmonic vibration, or the like, forecasting or analysis of earthquakes, and the like.

More specifically, a mother wavelet is generated from a part of a target signal (e.g., EEG) in simultaneous wavelet analysis. A fractal characteristic results from use of a mother wavelet derived from the signal itself, such that artifact (motion artifact or the like) can be reduced.

In one embodiment, step B) in the present invention further comprises subjecting the wavelet transformed signal to convolution processing into the signal data before the transform. Convolution processing is a two term computation that superimposes and adds function g to function f by translating function g. In such a case, convolution of functions f and g is denoted as f*g as the convolution computation, and is defined as follows.

[Numeral 3]

(f*g)(t)=∫f(τ)g(t-τ)dτ

The range of integration is dependent on the defined region of a function. Generally, a function defined with an interval of (- ∞, + ∞) is often used, so that calculation is often performed with a range of integration of - ∞ to + ∞. If on the other hand f and g are defined only in a finite interval, calculation is performed while presuming f and g as a periodic function (cyclic convolution) so that g(t-r) is within the defined region. Convolution for functions defined by discrete values is similarly defined using the total sum instead of integral. The range of total sum is also dependent on the defined region of the function. If a function is only defined in a finite interval, the function is deemed a periodic function to perform convolution computation. Any of the above can be used in the present invention. For a discrete system, convolution is often performed with a function redefined so that a value outside of the defined region is 0. This is referred to as linear (straight line) convolution. Since a discrete system uses the total sum without using an integral, such convolution is not referred to as a convolution integral, but convolution sum.

In one embodiment, the correlation processing in the present invention (including cross-correlation processing and autocorrelation processing) comprises creating a time variation wavelet, normalization of the signal, and convolution of the normalized signal. In this regard, "time variation wavelet (analysis)" refers to a wavelet created differently for each time frame, and refers to creating a mother wavelet and correlating with a signal for each sampled time segment.

The time frame of a mother wavelet can be appropriately determined by those skilled in the art depending on the sampling frequency when practicing the invention. For example, a mother wavelet is generated in a time frame of 10 to 120 seconds (or about 10 to 30 seconds) in an embodiment of a brainwave used when identifying pain. A specific example of a time frame includes, but is not limited to, at least about 20 seconds. Although not wishing to be bound by any theory, this is because it has been found that self-similarity can be readily created by using a mother wavelet using a time frame of at least 20 seconds, particularly for a biological signal such as a brainwave.

In one specific embodiment, the correlation processing (including cross-correlation processing and autocorrelation processing) of the invention can be performed using the following simultaneous wavelet transform. Specifically, step B can comprise: b-1) sampling a segment of the signal; b-2) generating a mother wavelet from the signal; b-3) optionally analyzing a correlation with the signal by extending and contracting the mother wavelet; and b-4) repeating b-1) to b-3) until a portion necessary for analysis of the signal is analyzed.

Such a methodology can be materialized by applying an instantaneous analysis method. An embodiment thereof is exemplified below.

The instantaneous analysis method performed in the present invention uses a part of a signal subjected to analysis as a mother wavelet. This allows calculation of similarity (especially self-similarity). A fractal characteristic results from using a real signal mother wavelet, so that various artifacts can be reduced. A mother wavelet is updated as the analysis window changes in the temporal axis. A time frame can be appropriately determined by those skilled in the art in accordance with the sampling frequency in light of the content herein. For example, the time frame can be set to 10 seconds to 60 seconds (or 1 to 5 minutes or the like) for brainwaves, but this is not a limiting example. Since the coarseness of signals subjected to analysis and basis vary depending on the sampling frequency, an embodiment of creating a basis with a signal in a high frequency band and extending this to find the correlation is advantageous in that a more accurate scalar product result can be obtained, but the present invention is not limited thereto. In addition, a signal from extraction of a signal is defined as the function $\Psi_{SIGNAL}(t)$. This function needs to satisfy the following conditions 1) a wavelet needs to have limited energy:

[Numeral 4]

$$E = \int_{-\infty}^{\infty} |\psi_{SIGNAL}(t)|^2 dt \ < \infty$$

variable E is the square of integrated amplitude. 2)

[Numeral 5]

$$\hat{\psi}_{SIGNAL}(f)$$

is Fourier transform $\Psi_{SIGNAL}(t)$ and needs to satisfy the following condition:

[Numeral] 6

$$C_g = \int_0^{\infty} \frac{\left|\hat{\psi}_{SIGNAL}(t)\right|^2}{f} df \ < \infty$$

A wavelet having an admissible constant satisfying $0 < C_g < \infty$ is referred to as an admissible wavelet. Traditionally, constant $C_g$ is referred to as a wavelet admissible constant. An admissible wavelet is

[Numeral 7]

$$\hat{\psi}_{SIGNAL}(t) = 0$$

,

and the integration result would be 0. For conventional wavelets, a basis function was used as an analyzing wavelet (AW), but a part of an actual signal is used as AW in instantaneous correlation function (ICF) analysis in the present invention.

ICF analysis is specified as

[Numeral 8]

$$ICF(t,a) = k_a \int_{-L_a/2}^{L_a/2} s(\tau,a) f(t+\tau)\, d\tau$$

',

wherein $s(\tau, b)$ is a part of an actual signal used as AW, $L_a$ is the length of a window function, $k_a$ is a standardization parameter, and $a$ and $\tau$ are scale and shift parameters. When a part of the observed signal (e.g., EEG or the like) is understood to be $s(\tau, b)$, self-similarity between an analyzing wavelet and observed signal can be analyzed. Similarity between $s(\tau, a)$ obtained from each signal can be detected in analysis of two signals. This analysis method can be applied to analysis of the observed signal with a basic frequency and harmonic component. Therefore, when a signal having a harmonic structure (e.g., nervous signal, brainwave, or the like) is analyzed, characteristics related to the basic frequency and harmonic component can be simultaneously detected. Thus, a change in a characteristic with a harmonic structure can be identified. In addition, directly performing instantaneous correlation analysis without preprocessing enabled real-time analysis in the truest sense in the present invention. In instantaneous correlation analysis, a wavelet with predetermined self-similarity for normalization of a basic basis is prepared, and the scalar product is calculated to find the instantaneous correlation.

In one embodiment in the present invention, brainwaves can be targeted. When using a brainwave, the signal comprises at least one signal calculated in a frequency band of $\delta$, $\theta$, $\alpha$, $\beta$, and $\gamma$ and four electrodes.

The feature and the coefficient are associated so that the level of the stimulation can be differentiated in the best manner by sigmoid fitting or a multiple regression model.

In one aspect, the present invention provides an apparatus for processing a signal of an object, comprising: A) a signal obtaining unit for obtaining a signal from an object; B) a processing unit for applying cross-correlation processing to the signal using a part or all of the signal; and C) a calculation unit for calculating a feature of the signal and a coefficient associated with the stimulation from a result of processing obtained in B).

An explanation is provided using Figure 19. Figure **19** describes a schematic diagram of the apparatus of the invention. A signal obtaining unit **100,** a processing unit **200,** and a calculation unit **300** indicate important constituent parts in the present invention. The apparatus can optionally comprise a display unit **400** and/or a differentiation unit **500.** A processing unit can comprise a storage unit **600** for storing processing results.

The signal obtaining unit **100** falls under (A), and any means that can obtain (e.g., measure) signals such as brainwaves (e.g., reaction to stimulation) can be used. Examples thereof include a brainwave meter, a seismometer, a vibrometer, an acoustic measurement instrument, and the like.

Any unit can be used as the processing unit **200** in the apparatus of the invention, as long as the unit is configured to be capable of cross-correlation processing (e.g., autocorrelation processing). Generally, a CPU or the like implemented with a program or the like can be used, but this is not a limiting example. Any program can be implemented, as long as the program performs cross-correlation processing (e.g., autocorrelation processing) on a signal obtained at a signal obtaining unit using some or all of the signal. A detailed embodiment thereof is described in detail in the section of (Simultaneous cross-correlation signal processing). The embodiment can be practiced by appropriately combining one or more characteristics thereof. The processing unit **200** performs the step of applying cross-correlation processing (e.g., autocorrelation processing) to using a part or all of the signal. The processing unit performs a series of calculations for performing cross-correlation processing (e.g., autocorrelation processing) using a mother wavelet generated by deriving a real signal mother wavelet (RMW) from an obtained signal or the like. The processing unit can be configured to allow a choice to first store obtained data and perform online processing subsequently. When performed in real time, an instantaneous correlation method is used.

The calculation unit **300** for calculating a feature of the signal and a coefficient associated with the stimulation from a result of processing can have any configuration, as long as a feature and a coefficient can be calculated from transformed data obtained at the processing unit. A plot diagram is created using data and fitted to a certain function pattern. Fitting to a function pattern can be performed using any methodology that is known in the art. The processing unit **200** and the calculation unit **300** can be materialized by the same unit (e.g., CPU) or configured separately.

In one aspect, the present invention provides a program for making a computer perform a method of processing a signal of an object in response to stimulation. In this regard, the method implemented with the program comprises: a) obtaining a signal in response to stimulation from the object; b) applying cross-correlation processing (e.g., autocorrelation processing) to the signal using a part or all of the signal; and c) calculating a feature of the signal and a coefficient associated with the stimulation from a result of processing obtained in b). A detailed embodiment is described in detail in the section of (Simultaneous cross-correlation signal processing). The embodiment can be practiced by appropriately combining one or more characteristics thereof.

The present invention also provides a recording medium for storing a program for making a computer perform a method of processing a signal of an object in response to stimulation. The method comprises: a) obtaining a signal in response to stimulation from the object; b) applying cross-correlation processing (e.g., autocorrelation processing) to the signal using a part or all of the signal; and c) calculating a feature of the signal and a coefficient associated with the stimulation from a result of processing obtained in b). A detailed embodiment is described in detail in the section of (Simultaneous cross-correlation signal processing). The embodiment can be practiced by appropriately combining one or more characteristics thereof.

Simultaneous signal processing is described using the following schematic diagram (Figure **18**).

**S1** is a step of taking in a signal subjected to measurement. This step can be materialized using an electroencephalograph or the like. For brainwaves, such brainwave data can be obtained using any methodology that is well known in the art. Brainwave data can be obtained by measuring an electrical signal of a brainwave and displayed by potential (can be displayed by $\mu V$ or the like) as amplitude data or the like. Frequency properties are displayed as power spectrum density or the like. Other signals can be obtained by using a suitable signal obtaining apparatus (seismometer, vibrometer, or the like).

In a preferred embodiment, brainwave data is preferably collected by a simple method, which can 1) use fewest possible number of electrodes (about two), 2) avoid the scalp with hair as much as possible, and 3) record while sleeping, to carry out the invention, but the number of electrodes can be increased as needed (e.g., can be 3, 4, 5, or the like).

**S2** is a part of a step for applying cross-correlation processing (e.g., autocorrelation processing) using a part or all of the signal. Specific example includes deriving a Real-signal Mother Wavelet (RMW) from a signal taken in.

**S3** is a step of determining whether to perform real time analysis. If so, the procedure proceeds to **S4,** and if not, data can be recorded **(S33)**. In such a case, offline analysis can be performed separately **(S35)**. Offline analysis can perform the same processing as online analysis in substantially the same manner as **S4.**

**S4** performs an instantaneous correlation method. This is a step for performing cross-correlation processing (e.g., autocorrelation processing) using a mother wavelet generated by deriving a real signal mother wavelet (RMW) from a signal taken in or the like.

**S5** is a step for performing brainwave analysis (e.g., analysis on pain) based on a result correlated thereby. Optionally, analysis result can be displayed or further differentiation (determination of further need for an analgesic or the like) can be performed **(S6).**

When performing brainwave analysis, a plot diagram is created using brainwave data and fitted to a function pattern. Fitting to a function pattern can be performed using any methodology that is known in the art. Specific examples of such fitting functions include, but are not limited to, a Boltzmann function, double Boltzmann function, Hill function, logistic dose response, sigmoid Richards function, sigmoid Weibull function, and the like. A standard logistic function is particularly called a sigmoid function. A standard function or a modified form thereof is common and preferred. When a regression coefficient for fitting to a function pattern is equal to or greater than a predetermined value, a threshold value can be calculated to separate pain levels into at least two or more levels (or pain levels into two or three or more quantitative/qualitative levels) based on a curve. When there are a plurality of features, a differentiation value used in differentiation of pain levels or a differentiation model parameter (regression coefficient or the like) can be calculated by using individual features alone for fitting to a function pattern or for model creation without consolidating all the features by averaging or the like. In such a case, evaluation such as what feature is more effective to be used can be performed based on ranking of the features by comparing coefficients or differentiation accuracy.

The present invention has an advantage in that pain can be analyzed without applying stimulation that is highly invasive to an objective upon measurement.

Some or all of the constituent elements of the apparatus in each of the embodiments described above can be comprised of a single system LSI (Large Scale Integration). For example, an apparatus **1000** can be comprised of a system LSI having the signal obtaining unit **100,** processing unit **200,** and calculation unit **300,** and optionally the display unit **400,** differentiation unit **500,** and storage unit **600.**

System LSI is ultra-multifunctional LSI manufactured by integrating a plurality of constituents on a single chip, or specifically a computer system comprised of a microprocessor, ROM (Read Only Memory), RAM (Random Access Memory), and the like. A computer program is stored in a ROM. The system LSI accomplishes its function by the microprocessor operating in accordance with the computer program.

The term system LSI is used herein, but the term IC, LSI, super LSI, and ultra LSI can also be used depending on the difference in the degree of integration. The methodology for forming an integrated circuit is not limited to LSI. An integrated circuit can be materialized with a dedicated circuit or universal processor. After the manufacture of LSI, a programmable FPGA (Field Programmable Gate Array) or reconfigurable processor which allows reconfiguration of the connection or setting of circuit cells inside the LSI can be utilized.

If a technology of integrated circuits that replaces LSI by advances in semiconductor technologies or other derivative technologies becomes available, functional blocks can obviously be integrated using such technologies. Application of biotechnology or the like is also a possibility.

One embodiment of the invention can be not only such an apparatus, but also a measurement, analysis, or processing method using characteristic constituent units contained in an apparatus as steps. Further, one embodiment of the invention can be a computer program for making a computer execute each characteristic step in various methods. One embodiment of the invention can also be a computer readable non-transient recording medium on which such a computer program is recorded.

In each of the embodiments described above, each constituent element can be materialized by being configured with a dedicated hardware or by executing software program that is suited to each constituent element. Each constituent element can be materialized by a program execution unit such as a CPU or a processor reading out and executing a software program recorded on a recording medium such as a hard disk or semiconductor memory. In this regard, software materializing the apparatus of each of the embodiments described above or the like can be a program such as those described above herein.

(Analysis technology)

In one aspect, the present invention provides a method of analyzing an object, comprising: analyzing a reaction to the stimulation of the object using the feature and the coefficient obtained by any methodology described herein in (Simultaneous cross-correlation signal processing). Each of the steps, features, and coefficients can use one or a combination of any embodiments described in (Simultaneous cross-correlation signal processing). In a preferred embodiment, the reaction subjected to analysis comprises pain.

In the context of Figure 18, this analysis technology performs the steps up to **S5** and optionally **S6.**

In one aspect, the present invention provides an apparatus for analyzing an object, comprising: A) a signal obtaining unit for obtaining a signal from an object; B) a processing unit for applying cross-correlation processing (e.g., autocorrelation processing) to the signal using a part or all of the signal; C) a calculation unit for calculating a feature of the signal and a coefficient associated with the stimulation from a result of processing obtained in B); and D) an analysis unit for analyzing a characteristic of the object using the feature and the coefficient.

In one aspect, the present invention provides a program for making a computer perform a method of analyzing an object. In this regard, the method comprises: a) obtaining a signal in response to stimulation from the object; b) applying cross-correlation processing (e.g., autocorrelation processing) to the signal using a part or all of the signal; c) calculating a feature of the signal and a coefficient associated with the stimulation from a result of processing obtained in b); and d) analyzing a reaction to the stimulation of the object using the feature and the coefficient. Each of the steps, features, and coefficients can use one or a combination of any embodiments described in (Simultaneous cross-correlation signal processing). In a preferred embodiment, the reaction subjected to analysis comprises pain. In the context of Figure **18,** this analysis technology performs the steps up to **S5** and optionally **S6.**

In one aspect, the present invention provides a recording medium for storing a program for making a computer perform a method of analyzing an object. The method implemented by the stored program comprises: a) obtaining a signal in response to stimulation from the object; b) applying cross-correlation processing (e.g., autocorrelation processing) to the signal using a part or all of the signal; c) calculating a feature of the signal and a coefficient associated with the stimulation from a result of processing obtained in b); and d) analyzing a reaction to the stimulation of the object using the feature and the coefficient. Each of the steps, features, and coefficients can use one or a combination of any embodiments described in (Simultaneous cross-correlation signal processing). In a preferred embodiment, the reaction subjected to analysis comprises pain. In the context of Figure **18,** this analysis technology performs the steps up to **S5** and optionally **S6.**

Various analysis methodologies used in an analysis technology are conceivable.

For example, signals after wavelet transform are extracted as a feature such as a mean value or other representative value, and the relationship with pain is fitted to a sigmoid curve to generate a threshold value or determination value for pain classification. Optionally, relative pain levels can be differentiated using the determination value or threshold value after calibration of the determination value or threshold value. The relative pain magnitude from a plurality of brainwave measurements can be estimated to estimate a pain level, based on the relative relationship of brainwave amplitude, frequency power, or the real signal wavelet feature in the invention and pain. The presence of a certain relative relationship between brainwave amplitude or frequency property and pain is a phenomenon elucidated by the inventors, which is also applicable for the real signal wavelet feature of the invention. The present invention can classify the magnitude and level of pain without using the magnitude of pain reported by an object being estimated, and objectively and accurately classify pain of the object being estimated by further fitting the relative relationship to a sigmoid curve. Furthermore, the quality of pain such as "unbearable" pain, "bearable" pain, and "comfortable pain" can be classified, so that a therapeutic effect can be more accuracy evaluated. For the accurate classification, the effect of noise was significantly reduced in cases with wavelet transform from cases without wavelet transform to enable precise diagnosis. The accuracy improves 10% or more.

As used herein, "sigmoid function" or "sigmoid curve" refers to a real function exhibiting a sigmoidal shape. In the present invention, standardized or normalized subjective pain intensity and standardized or normalized EEG amplitude can be used to generate a sigmoid curve.

A sigmoid function is generally represented by $\sigma_a (x) = 1/(1 + e^{-ax}) = (\tanh (ax + 2) + 1)/2$.

A decreasing sigmoid function can be expressed by subtraction from 1 or a reference value. A monotonically increasing continuous function of $(-\infty, \infty) \rightarrow (0, 1)$ has one inflection point. This function has an asymptote at $y = 0$ and $y = 1$. In such a case, the inflection point is $(0, 1/2)$. For setting an asymptote, the function may not have an asymptote at 0 or 1 depending on the measured (and optionally normalized) amplitude data (EEG amplitude), but the maximum value and minimum value can be used as an asymptote in such a case.

As used herein, "fitting" to a sigmoid function refers to a technique of fitting measured values or a curve obtained therefrom to approximate a sigmoid, which can be performed based on any approach. For example, a known sigmoid fitting function can be used. Examples of such fitting include least square fitting, nonlinear regression fitting (MATLAB's nlinfit function or the like), and the like. After fitting, a regression coefficient can be calculated for the approximated sigmoid curve to determine whether the sigmoid curve can be used or preferable in the present invention. For a regression coefficient, a regression equation model is effective. The adjusted coefficient of determination ($R^2$) is desirable with a numerical value closer to "1" such as 0.5 or greater, 0.6 or greater, 0.7 or greater, 0.8 or greater, 0.85 or greater, 0.9 or greater, or the like. A higher numerical value has higher confidence. The differentiation accuracy of a fitting function can be studied by using a specific threshold value to categorize and compare an estimated value and an actual measurement value (this is referred to as differentiation accuracy in the analysis of the invention). Although not wishing to be bound by any theory, a high degree of fitting to a sigmoid function is not necessarily the same as a high degree of differentiation of pain levels using the same. For example, if asymptotes for weak and strong levels are close and variability of both data is high, it is understood that accuracy of differentiating weak and strong would not be high even if sigmoid function is high approximated and β coefficient is high.

As used herein, "calibration", for a pain classifier, refers to any process for correcting the pain classifier or a corrected value thereof generated by fitting to a sigmoid function more in line with the classification of the object of measurement. Examples of such calibration include a weighting methodology to increase/decrease a value so that the classification of a pain level is maximized and the like. Other examples include, but are not limited to, methodologies such as a method of applying a specific reference stimulation at a specific time interval and weighting using a coefficient or the like, or correcting a coefficient in an existing model (e.g., multiple regression or logistic model), from the change in the amount of brain activity to correct the differentiation of a change in pain within an individual.

For example, the plurality of brainwave data comprises a sufficient number of brainwave data to enable fitting to a sigmoid curve. For such fitting, brainwave data for, for example, at least three stimulation intensities, preferably four, five, six, or seven or more stimulation intensities can be used.

When using a sigmoid curve, once such fitting is completed, a regression coefficient is optionally evaluated to determine whether the fitting is appropriate. Generally, 0.5 or greater, or preferably 0.6 or greater can be used as a threshold value of a regression coefficient. If a suitable regression coefficient is accomplished, the fitting is suitable, so that analysis proceeds to the next analysis. If a regression coefficient is evaluated to be unsuitable, additional brainwave data can be obtained for re-fitting to a sigmoid curve with existing data, or brainwave data can be obtained again for re-fitting to a sigmoid curve only with the newly obtained data.

Once fitting is completed, a determination value or threshold value is generated based on a sigmoid curve. A determination value or threshold value refers to a feature (e.g., mean value, representative value, maximum value, or the like associated with stimulation in a certain time segment) extracted from amplitude data or frequency data from a brainwave or the real signal wavelet transform data, for classifying pain into at least two or more classes. For example, this can be a value for classifying pain into "weak pain" and "strong pain". It is desirable that this value can clinically classify "unbearable pain requiring therapy" and "bearable pain not requiring therapy". Such a determination value or threshold value can be determined by referring to an inflection point. A determination value or threshold value that is generated can be used directly, but the value can be optionally calibrated. For example, calibration is materialized, when classifying pain into "weak pain" and "strong pain", by fitting the value to actually obtained amplitude data or derivative feature (including real signal wavelets) and changing the value to a value with less deviation (i.e., classification into a different class, including, for example, determination as strong pain when it should be weak pain or vice versa) or to a value with the least deviation.

When an increase in pain is associated with increase in a wavelet feature for brainwave data subjected to wavelet transform, (i) first pain corresponding to first brainwave data can be estimated to be greater than second pain corresponding to second brainwave data, which is different from the first brainwave data, if the amplitude of the first brainwave data (strength of cross-correlation in this case) is greater than the amplitude of the second brainwave data and (ii) the first pain can be estimated to be less than the second pain if the amplitude of the first brainwave data is less than the amplitude of the second brainwave data. In such a case, beyond the relative difference in pain, the data can be further compared to a pain classifier for separating weak and strong pain to classify which level of pain (e.g., strong pain, weak pain, or the like) the first and second pain falls under. For example, more detailed differentiation is possible such as whether first and second pain are relatively different while being within the strong level range, or whether the pains have different weakness while being within a weak level. Thus, the change in the level of magnitude of pain before, during, and/or after therapy (e.g., "still strong pain", "pain transitioned from a strong to weak level", or the like) can be examined to evaluate the therapeutic effect by, for example, measuring brainwave data before and after therapy and extracting a feature comprising a real signal wavelet.

For example, additional brainwave data may be obtained for the plurality of brainwave data.

A certain embodiment provides a method and an apparatus for generating a determination value or a threshold value for classifying pain of an object based on wavelet transformed brainwave data of the object, and a method and apparatus for analysis using the same. In this regard, the object being estimated is stimulated with a plurality of levels of stimulation intensity. The number of stimulation intensity levels (magnitudes) provided is a sufficient number that can be fitted to a sigmoid function pattern. For fitting to a sigmoid function pattern, at least two, preferably at least three, preferably at least four, preferably at least five, preferably at least six, or more levels (magnitude) of stimulation intensity can be used. As differentiation means or differentiation instrument, a multiple regression model, machine learning model (e.g., SVM), or the like can also be used besides sigmoid functions. In such a case, brainwave data associated with a pain level to be differentiated/estimated and derivative features are desirably a greater data set comprising a plurality of levels.

These comprehensive or specific embodiments can be materialized with a system, method, integrated circuit, computer program, or a storage medium such as a computer readable CD-ROM or any combination of a system, method, integrated circuit, computer program, and storage medium.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present invention has been described while showing preferred embodiments to facilitate understanding. The present invention is described hereinafter based on Examples. The above descriptions and the following Examples are not provided to limit the present invention, but for the sole purpose of exemplification. Thus, the scope of the present invention is not limited to the embodiments or the Examples specifically described herein and is limited only by the scope of claims.

[Examples]

Examples are described hereinafter. The objects used in the following Examples were handled, as needed, in compliance with the standards set forth by the Osaka University and Hiroshima City University, and the Declaration of Helsinki and ICH-GCP in relation to clinical studies.

(Example 1: Wavelet transform of biological signal)

This Example performed wavelet transformation of a biological signal.

(Biological signal)

The inventors measured $SpO_2$, pulse waves, skin conductance, and EEG. This section describes each characteristic of biological signals.

(1. Skin conductance)

Skin conductance is also known as galvanic skin response (GSR) or skin potential (EDA). The latter refers to electrical change measured on the skin surface that is generated when the skin receives a neurarchy signal from the brain (Argyle. N, "Skin Conductance Levels in Panic Disorder and Depression.", The Journal of Nervous and Mental Disease, 179, 563-566, 1991". When humans experience activation of an emotion or increase in labor, the brain sends a signal to the skin to increase the perspiration level. Since sweat contains water and electrolytes, the electrical conductance of the skin significantly increases to a measurable level.

(2. $SpO_2$)

The oxygen saturation level is expressed as the level of hemoglobin contained in red blood cells bound to an oxygen molecule. A pulse oximeter measures the hemoglobin level and then calculates the mean saturation percentage ($SpO_2$), thus indirectly measuring the oxygen saturation level (Mary Jo Grap. "Pulse Oximetry," Critical Care Nurse. 22, 69-74, 2002). Such a noninvasive process involves insertion of a finger into a device in which a red light measures the reddening of blood pulsating at the finger.

(3. Pulse wave)

A pulse wave is a waveform that changes in accordance with the volume of a blood vessel induced by influx of blood (K. Tanaka, "Perfusion Index and Pleth Variability Index", The Journal of Japan Society for Clinical Anesthesia, Vol.31, No.2, 2011). While the arterial system is a direct traveling wave moving towards the heart, the venous system is a reflected wave. Systolic and diastolic phases can be determined from a pulse waveform. The inventors can draw a conclusion related to the interaction between the heart and the arterial system therefrom. Pulse waves were continuously and invasively measured with a pulse oximeter.

(4. Brainwave)

EEG is a record of electrical activity of the brain from the scalp. Event related potential (ERP) is a measured brain response, which is a direct result of specific sensation, cognition, or instance of motion (American Electroencephalographic Society 1991 Guidelines for standard electrode position nomenclature. Journal of Clinical Nurophysiology, 8, 200-202). The inventors measured ERP induced by distressing temperature stimulation. A small metal disk (electrode) covered with a silver chloride coating is placed at special sites on the skin. These positions are identified using the international 10/20 system. The essence of the system is in the difference in the percentage in the 10/20 range between the nasion-inion line and a fixed point.

(Analysis and characteristic extraction)

The EEG characteristic was extracted using a time-frequency analysis. For the pulse wave characteristic, heart rate variability (HRV) analysis was performed to obtain the correlation between pulse waves and heart rate.

(LF/HF ratio)

Power spectrum analysis of the heart rate variability or cardiac cycle (R-R interval) has been widely used for quantification of the cardiac autoregulation (K. Oue, S. Ishimitsu, "Evaluating Emotional Responses to Sound Impressions Using Heart Rate Variability Analysis Of Heart Sound", Tenth International Conference on Innovative Computing, Information and Control, pp.118, Dalian, China, 2015). This method sorts the total change in a series of continuous pulse into its frequency components and identifies two main peaks: low frequency (LF) of less than 0.15 Hz and high frequency (HF) of 0.15 to 0.4 Hz. A HF peak reflects the parasympathetic activity of the heart. In the same method, an LF peak has a sympathetic and parasympathetic components. The ratio of LF to HF (LF/HF) is used to quantify the relationship between the sympathetic activity and parasympathetic activity based thereon.

(Wavelet transform)

Next, the wavelet coefficient of continuous wavelet transform is given by

[Numeral 9]

$$T(a,b) = \frac{1}{\sqrt{a}} \int_{-\infty}^{\infty} x(t)\psi^* \left(-\frac{b-t}{a}\right) dt$$

wherein x(t) represents a time domain signal of EEG, and a and b are expansion and translation parameters, respectively. * indicates a complex conjugate. Parameter a correlates with frequency, and b correlates with time. A Morlet wavelet was used as the mother wavelet Ψ(t). The wavelet is subsequently given by

[Numeral 10]

$$\psi(t) = e^{-t^2/2}\cos 5t$$

Parameters a and b are associated with translation and expansion of the mother wavelet, respectively (Paul S. Addison, "The Illustrated Wavelet Transform Handbook: Introductory Theory and Applications in Science, Engineering, Medicine and Finance", CRC Press, July 15, 2002).

(Simultaneous wavelet analysis)

A mother wavelet was generated from a part of EEG. This analysis mainly targeted self-similarity of biological signals. Artifacts such as motion artifacts can be reduced with a fractal characteristic, which becomes prominent by the use of an EEG mother wavelet. Wavelet transform is performed in a short period of time. As the analysis window moves with respect to the temporal axis, the mother wavelet is updated continuously. An EEG extraction signal is defined as the function $\Psi_{EEG}(t)$. This function needs to satisfy the following conditions. 1) A wavelet needs to have limited energy:

[Numeral 11]

$$E = \int_{-\infty}^{\infty} |\psi_{EEG}(t)|^2 dt < \infty$$

Variable E is the square of integrated amplitude. 2)

[Numeral 12]

$$\hat{\psi}_{EEG}(f)$$

is Fourier transform $\Psi_{EEG}(t)$ and needs to satisfy the following condition:

[Numeral 13]

$$C_g = \int_0^{\infty} \frac{\left|\hat{\psi}_{EEG}(t)\right|^2}{f} df < \infty$$

A wavelet having an admissible constant satisfying $0 < C_g < \infty$ is referred to as an admissible wavelet. Traditionally, constant $C_g$ is referred to as a wavelet admissible constant. An admissible wavelet is

[Numeral 14]

$$\hat{\psi}_{EEG}(t) = 0,$$

and integration results in 0. For conventional wavelets, a basis function was used as an analyzing wavelet (AW). A part of an actual signal was used as AW in instantaneous correlation function (ICF) analysis in this Example.

ICF analysis is specified as

[Numeral 15]

$$\mathrm{ICF}(t,a) = k_a \int_{-L_a/2}^{L_a/2} s(\tau,a)f(t+\tau)\,d\tau$$

wherein $s(\tau, b)$ is a part of an actual signal used as AW, $L_a$ is the length of a window function, $k_a$ is a standardization parameter, and $a$ and $\tau$ are scale and shift parameters, respectively. When a part of the observed signal is understood to be $s(\tau, b)$, self-similarity between an analyzing wavelet and observed signal can be analyzed. Similarity between $s(\tau, a)$ obtained from each signal can be detected in analysis of two signals. This technique can be applied to analysis of the observed signal with a basic frequency and harmonic component. Therefore, when a signal having a harmonic structure is analyzed, characteristics related to basic frequency and harmonic component can be simultaneously detected. Thus, a change in a characteristic with a harmonic structure is expected to be identifiable (S. Ishimitsu, H. Kobayashi., "Study on Instantaneous Correlation Analyses of Acceleration Car Interior Noise using Wavelets and its Subjective Evaluation", Transactions of the Japan Society of Mechanical Engineers, serise C, Vol. 72, No. 719, pp.2094-2100 (2006); H. Ishii, H. Uemura, Z. Zhang, T. Imamura., "Development of identi cation for noise source using visualization of sound and vibration", Transactions of the Society of Instrument and Control Engineers, Vol. 10, No. 9, pp. 73-80 (2011); Z. Zhang, H. Ikeuchi, H. Ishii, H. Horihata, T. Imamura, T. Miyake., "Real-Signal Mother Wavelet and Its Application on Detection of Abnormal Signal: Designing Average Complex Real-Signal Mother Wavelet and Its Application", Transactions of the Society of Instrument and Control Engineers, Vol. 10, No. 9, pp. 73-80 (2011)).

(Experimental procedure)

This section describe the experimental induction of pain by temperature stimulation (Y. Shimazaki, A. Yoshida, S. Sato, S. Nozu, "A Study of Hyperthermia Sensitivity on Human Body based on Local Thermal Load", Japanese society of Human-Environment System 34 in Niigata, November, 2010). The inventors estimated objective pain from various biological signals. Temperature stimulation was applied to the forearm of a subject to cause pain. The inventors measured the change in vital signs induced by the stimulation. The temperature stimulation was induced by a metal probe fixed onto the forearm by using a band. While the subject was sitting, distressing temperature stimulation and normal temperature stimulation (equal to the body temperature of the subject) were alternatingly applied. Although temperature stimulation needs to be sufficiently high to cause pain, there is an individual difference in the temperature which is felt by a subject as distressing in accordance with the sensitivity to pain of the subject. To solve this problem, subjects reported a threshold value for judging a temperature of temperature stimulation as distressful. The inventors monitored $SpO_2$, pulse waves, skin conductance, and EEG.

(Experimental results)

While the experiment was conducted using more than 20 subjects, only a part of all data is described herein. Figure **1** shows a temperature stimulation pattern and VAS. The temperature stimulation and VAS values had similar waveforms.

(All biological signals)

Figure **2** shows all the biological signals: temperature stimulation, $SpO_2$, skin conductance, pulse wave, and EEG. It is apparent that skin conductance is inversely proportional to the temperature stimulation pattern. Since the sympathetic nervous system of the autonomic nervous system is stimulated strongly, the activity of the sweat glands increases, and the skin conductance increases. The inventors revealed the relationship between pain, EEG, and pulse waves by extracting a characteristic from a waveform.

(Results of pulse wave)

Figure **3** shows the R-R interval frequency observed while a subject is being stimulated and while the subject is not stimulated. The frequency was obtained from the R-R interval over 60 seconds during application of stimulation. HF/LF involving pain exhibited a value of 59, which was greater compared to a period of no pain. Furthermore, the LF component decreased, and the HF component increased. It is inferred that the sympathetic nervous system is activated by the temperature stimulation based on these observations. The result suggests that HF/LF increases with temperature, so that HF/LF is a pain indicator.

(Result of brainwaves)

An EEG signal was filtered to select an $\alpha$ wave band frequency without introducing additional noise before analysis. Figure **4** shows the frequency extracted from an EEG signal: temperature stimulation, amplitude (power) Fourier transform, wavelet transform, and proposed wavelet transform. A characteristic is the total of the numerical values of each scale obtained by each transform. All characteristics obtained by EEG tended to increase in the pain free period, but the characteristics increased with pain in some cases. Since the proposed wavelet transform does not increase compared to other characteristics while a subject is receiving stimulation involving distress, a characteristic obtained by the proposed wavelet transform tends to be more convincing. Noise was reduced using the self-similarity of EEG. It is conjectured that such a result was reached because proposed wavelet coefficients are not involved with the amplitude of a signal but waveform.

(Preliminary experimental result)

Figure **5** shows results when using a stimulation free brainwave for the mother wavelet (MW). It can be understood that a higher numerical value is detected in a time frame with high correlation with MW. The natural world has self-similarity, which breaks down when unstable, so that it is judged that pain could be detected. With this findings, analysis thereof was conducted below in Example 2.

(Real time wavelet)

Figure **6** shows an example of model analysis in real-time wavelet analysis, based on the above findings. First, it is recommended that (1) a real signal segment is sampled, (2) a mother wavelet is generated from this signal, (3) optionally correlation with the signal is analyzed by extending and contracting (normalizing) the mother wavelet, and (1) to (3) is repeated until a portion necessary for analysis of the signal is analyzed to perform real signal wavelet analysis. Signals after transformation are shown in the bottom right diagram of Figure **6.** A local frequency component of a signal is extracted. This completes the calculation of a segment of signal T(a, b)

(Summary/conclusion)

In this Example, the inventors studied how a characteristic is extracted and how various biological signals are measured when a subject received contact temperature stimulation to objectively estimate pain. $SpO_2$ was not related to pain, but skin conductance decreased with an increase in pain. The inventors found that an EEG derived characteristic decreases with an increase in pain and skin conductance. In particular, a proposed wavelet coefficient has the most important characteristic as described above. Since the result is not affected by noise such as motion artifacts, it is suggested that the proposed wavelet coefficient is a robust characteristic. The effectiveness of the proposed wavelet transform with regard to EEG is evaluated by a recognition experiment using other characteristics in Example 2 and thereafter.

(Example 2: Differentiation of high temperature pain level using real signal wavelet characteristic)

This Example further analyzed EEG subjected to the wavelet transform performed in Example 1 using a high temperature stimulation paradigm. In particular, differentiation accuracy of two levels that are weak and strong pain was studied.

(Participants)

40 healthy adult subjects in their 20s to 70s participated in this Example. Informed consent was obtained from the participants prior to the clinical trial. All participants self-reported as having no history of a neurological and/or psychiatric illness, or acute and/or chronic pain under clinical drug therapy conditions. This Example was in compliance with the Declaration of Helsinki and conducted under approval of the Osaka University Hospital ethics committee.

(Procedure)

A temperature stimulation system (Pathway; Medoc Co., Ltd., Ramat Yishai, Israel) was used to apply high temperature pain stimulation from levels 1 to 6 to the inside right forearm of the participants (Figure 7) . The temperature was increased by 2°C from level 1 at 40°C to level 6 at 50°C, with a baseline temperature of 35°C. A trial block at each stimulation level consisted of three stimulations. Each stimulation had a plateau lasting 15 seconds, and a waiting period for increase and decrease of 5 seconds. There was a 5 second interval between stimulations. The rest between blocks was fixed at 100 seconds. The participants wore a thermal stimulation probe on the inside left forearm, and received thermal stimulation while lying down on an armchair. The participants continuously evaluated pain intensities in the range of 0 to 100 (0: "no pain"; 100: "unbearable pain") on a computerized visual analog scale (COVAS). COVAS data was simultaneously recorded with changes in stimulation intensities.

(Brainwave (EEG) data record)

Commercially available Bio-Amplifier (EEG 1200: Nihon Koden) was used to record EEG from four scalp Ag/AgCl scalp electrodes (Fz, Cz, C3, and C4). The frontmost electrode Fp1 was used to record EOG activity. Reference electrodes were attached to both earlobes, and an outside electrode was placed on the forehead. The sampling rate was set to 1000 Hz and amplified using a band pass filter in the range of 0.3 to 120 Hz. The impedance of all electrodes was less than 15 kΩ.

(EEG analysis)

Electrode data from Fz, Cz, C3, and C4 was used for analysis. A notch filter was applied first to continuous EEG data to remove ham noise (60 Hz). EOG artifacts were reduced based on the following regression filter.

$$[\text{Numeral 16}]$$
$$\text{Raw EEG} = \beta \times \text{EOG} + C$$
$$\text{EEG estimate} = \text{raw EEG} - \beta \times \text{EOG}$$

$\beta$: regression coefficient
C: intercept
EEG estimate: estimated EEG

Fp1 was the closest to the left eye and heavily affected by the eye movement, so that Fp1 data was used as EOG data.

(Simultaneous wavelet transform)

EEG was subjected to simultaneous wavelet transform processing in accordance with the procedure shown in Example 1. With a 20 second signal segment, mother wavelets was extended and contracted in 1 to 40 levels. For creating a mother wavelet, five frequency bands, i.e., $\delta$, $\theta$, $\alpha$, $\beta$, and $\gamma$, were subjected to transformation. To retrieve signals limited to each frequency band, the original amplitude data was subjected to Fourier transform, passed through a band pass filter that leaves only the corresponding frequency band, and returned to the original amplitude data. The amplitudes were converted into absolute values, and the entire signal was standardized with the maximum value. 20 second mother wavelet was sampled, subjected to a Blackman window function, and standardized with the absolute value of amplitude, and then the result was convoluted into EEG data while the length of the mother wavelet was extended and contracted in 1 to 40 levels to calculate the cross-correlation coefficient. This process was repeated in twice the time frame of the mother wavelet from the starting point of EEG data.

(Feature extraction)

15 seconds of data in the stimulation application segment from the starting point of each stimulation application was sampled (level 6 × 3 stimulations) . The values were converted to absolute values, and the mean value was calculated. The mean potential of levels 1 and 2 was used as the feature for weak pain level to be differentiated, and the mean value of levels 5 and 6 was used as the feature for strong pain level. A total of 160 samples (strong/weak level × two levels × 40 subjects) and 20 wavelet features (4 electrodes × 5 frequency bands) were inputted into differentiation analysis.

(Differentiation analysis using sigmoid function fitting)

Fitting to a sigmoid curve was performed based on signals from simultaneous wavelet transform processing. All 20 features were consolidated (averaged) into one.

(Results)

The results are shown in Figure **8.** 80 samples of weak pain level and 80 samples of strong pain level were continuously arranged for fitting with a sigmoid function. The approximation function is expressed by the following mathematical equation, and the goodness of fit was "β = 0.36, p < 0.0001".

[Numeral 17]

$$Y = 0.5327 - 0.1199/(1 + 10^{(80.4998 - X) \times 24.2645})$$

The pain differentiation value corresponding to the inflection point of the sigmoid approximation function was "0.4728". When 160 samples were differentiated with weak pain level as "> 0.4728" and strong pain level as "≤ 0.4728", the overall differentiation accuracy was "67.5%", thus materializing differentiation accuracy exceeding the chance level by about 20%.

As shown in Figure **9,** real signal wavelet and frequency power features were compared with respect to the difference in the modulation width involving the change from weak pain level to strong pain level of a sigmoid function. As can be understood from visual comparison of the alpha band and β band in Figure **10,** the modulation width is greater for real signal wavelets. In fact, when five frequency bands were compared using a paired t-test, a significant difference was found in δ to β bands (δ: t = 8.43, p < 0.0001; θ: t = 8.10, p < 0.0001; α: t = 6.663, p < 0.0001; β: t = 7.90, p < 0.0001), and a significant tendency was observed in the γ band (t = 1.90, p = 0.07) (Figure **11**).

It can be understood in view of the above results that a real signal wavelet feature has a more robust differentiation performance than a frequency power feature and a differentiation accuracy beyond the chance level.

(Example 3: Differentiation analysis 1 of electrical pain level using real signal wavelet characteristic)

This Example performed analysis on EEG subjected to wavelet transform performed in Example 1 using an electrical stimulation paradigm. In the same manner as Example 2, differentiation accuracy of two levels (weak/strong) of pain was compared and examined for cases where a wavelet feature was used and cases where a wavelet feature was not used.

(Participants)

41 healthy adult subjects in their 20s to 70s participated in this Example. Informed consent was obtained from the participants prior to the clinical trial. All participants self-reported as having no history of a neurological and/or psychiatric illness, or acute and/or chronic pain under clinical drug therapy conditions. This Example was in compliance with the Declaration of Helsinki and conducted under approval of the Osaka University Hospital ethics committee.

(Method)

Example 12 shows the outline of a method of the Example. Figure 7 shows the outline of the experimental method (experimental paradigm (electrical stimulation)) . Electrical stimulation intensities corresponding to weak, moderate, and strong pain levels were identified using a quantitative perception and pain sensation analyzer (PAINVISION CO., Ltd., Osaka, Japan) for each individual. Electrical stimulation intensity subjectively matching 10°C, 0°C, and -5°C in a low temperature stimulation paradigm performed concurrently was used for each individual as the standard for determining a pain level. Each stimulation was applied for 15 seconds at each level. Participants subjectively evaluated pain levels using COVAS in parallel with the application of stimulation. Differentiation analysis used level 1 (3 stimulations) as weak pain level and level 3 (3 stimulations) as strong pain level, and targeted a total of 246 samples for differentiation. For the features, mean amplitude (absolute value, standardized) for 15 seconds after application of stimulation, frequency power (4 electrodes × 5 frequency bands), and real signal wavelet (4 electrodes × 5 frequency bands) were used.

(Differentiation analysis using multiple regression model)

Differentiation accuracy was studied for cases where the following was inputted into a multiple regression model: (1) only mean amplitude and frequency features; and (2) a wavelet feature in addition to the features in (1). First, data was divided into learning and test data at a ratio of 8 to 2. The learning data was divided into 10 and 10-fold cross validation was performed to determine a partial regression coefficient of each feature and the intercept of a regression equation. The test data was differentiated/estimated using the parameters to calculate differentiation accuracy. This process was performed 1000 times to compare the difference in differentiation accuracy of (1) and (2).

(Results)

The mean value and standard deviation of partial regression coefficients (n = 1000) are shown in Figure **13** for cases using a real signal wavelet and cases not using a real signal wavelet. The feature coefficient in cases without a real signal wavelet is overall smaller. The absolute value of a coefficient was 1 or greater for only two features (Fz($\beta$) = -1.2619, Fz($\gamma$) = 2.6429) . Meanwhile, when a real signal wavelet was inputted, only two frequency features was 1 or greater (C4($\alpha$) = -1.5993, Fz($\gamma$) = 2.6064), but 9 out of 20 wavelet features exhibited 1 or greater (Fz($\delta$) = -2.5392, C4($\delta$) = 3.6528, Cz($\theta$) = -3.5916, C3($\alpha$) = -3.2487, Fz($\beta$) = - 3.6958, C3($\beta$) = 1.2981, C4($\beta$)= -5.2144, Fz($\gamma$) = 1.8334, C3($\gamma$) = 3.7275).

Figure **14** shows the differentiation accuracy for test data from 1000 runs. Differentiation accuracy without inputting a real signal wavelet was "52.5 $\pm$ 6.1%", which was different by only about 3 points from the differentiation accuracy of "49.8 $\pm$ 6.9%" for cases where differentiation labels were randomized. Meanwhile, the differentiation accuracy when a wavelet feature was inputted was "64.1 $\pm$ 6.4%", which was about a 12% improvement in accuracy. The above results suggest that wavelet features function more effectively than other features in differentiation of pain levels and are useful for automatic differentiation of pain levels.

(Example 4: Differentiation analysis 2 of electrical pain level using real signal wavelet characteristic)

This Example performed new differentiation analysis on EEG subjected to wavelet transform performed in Example 1 using an electrical stimulation paradigm used in Example 3. In the same manner as Example 3, differentiation accuracy of two levels (weak/strong) of pain was analyzed in cases using a wavelet feature and using minimally quantitatively/qualitatively corresponding frequency power.

(Participants)

41 healthy adult subjects in their 20s to 70s participated in this Example. Informed consent was obtained from the participants prior to the clinical trial. All participants self-reported as having no history of a neurological and/or psychiatric illness, or acute and/or chronic pain under clinical drug therapy conditions. This Example was in compliance with the Declaration of Helsinki and conducted under approval of the Osaka University Hospital ethics committee.

(Method)

Figure **15** shows the outline of a method of the Example. Figure **7** shows the outline of the experimental method. Electrical stimulation intensity corresponding to weak, moderate, and strong pain levels was identified using a quantitative perception and pain sensation analyzer (PAINVISION CO., Ltd., Osaka, Japan) for each individual. Electrical stimulation intensity subjectively matching 10°C, 0°C, and -5°C in a low temperature stimulation paradigm performed concurrently was used for each individual as the standard for determining a pain level. Each stimulation was applied for 15 seconds at each level. Participants subjectively evaluated pain levels using COVAS in parallel with the application of stimulation. Differentiation analysis used level 1 (3 stimulations) as weak pain level and level 3 (3 stimulations) as strong pain level, and targeted a total of 246 samples for differentiation. For the features, real signal wavelets for 15 seconds after application of stimulation (4 electrodes $\times$ 5 frequency bands) were used.

(Differentiation analysis using machine learning)

As a machine learning method, "Support Vector Machine Recursive Feature Elimination: SVM-RFE" (Guyon I, Weston J, Barnhill S, & Vapnik V. "Gene selection for cancer classification using Support Vector Machine." Machine Learning 46, 389-422, 2002) was used. Figure **16** shows the procedure. 246 samples of two levels of pain, i.e., weak and strong, were differentiated with 20 features. A radial basis (Gaussian) function was used as the kernel. First, the weighting coefficients of 20 features were calculated and ranked by a method of eliminating a feature exhibiting the lowest standard value in order (RFE). Next, leave-one-out cross validation was independently performed for cases of (1) real signal wavelet and (2) frequency power feature, by using SVM, to calculate and compare differentiation accuracy.

(Results)

Figure **17** shows the result of analysis. (A) shows ranking of wavelet features and the differentiation accuracy of leave-one-out cross validation when features were increased one at a time from the top ranking feature. When 12 features were used, differentiation accuracy was "71.1%", exhibiting accuracy that is 20% or more greater than the chance level. (B) shows results of ranking and differentiation accuracy of frequency power. When 15 features were used, the highest differentiation accuracy was "62.6%", but accuracy was about 10% lower compared to differentiation accuracy using a wavelet. In view of the above results, a wavelet feature can be considered as a "self-enhanced feature" that improves differentiation accuracy more than the sample type of frequency power. It can be concluded that the advantage thereof has been demonstrated through Examples 2 to 4.

(Note)

As disclosed above, the present invention has been exemplified by the use of its preferred embodiments. However, it is understood that the scope of the present invention should be interpreted based solely on the Claims. It is also understood that any patent, patent application, and references cited herein should be incorporated herein by reference in the same manner as the contents are specifically described herein. The present application claims priority to Japanese Patent Application No. 2017-148350 (filed on July 31, 2017). The entire content thereof is incorporated herein by reference.

[Industrial Applicability]

The present invention provides a technology that can readily and precisely classify events (e.g., pain) due to an abnormality in signals and accurately differentiate pain or the like, when it is not desirable or difficult to perform pre-processing. The present invention can diagnose or treat pain or other abnormalities in more detail.

[Reference Signs List]

| | |
|---|---|
| **100:** | signal obtaining unit |
| **200:** | processing unit |
| **300:** | calculation unit |
| **400:** | display unit |
| **500:** | differentiation unit |
| **600:** | storage unit |
| **1000:** | apparatus |

**Claims**

**1.** A method of processing a signal of an object in response to stimulation, comprising:

a) obtaining a signal in response to stimulation from the object;
b) applying cross-correlation processing to the signal using a part or all of the signal; and
c) calculating a feature of the signal and a coefficient associated with the stimulation from a result of processing obtained in b).

**2.** The method of claim 1, wherein the cross-correlation processing comprises autocorrelation processing.

**3.** The method of claim 1 or 2, wherein the correlation processing comprises finding self-similarity for each time.

**4.** The method of claim 1, wherein the signal does not have self-similarity or has a missing portion.

**5.** The method of claim 1, wherein the signal is a biological signal.

**6.** The method of claim 1, wherein the biological signal is a brain signal.

**7.** The method of claims 1 to 6, wherein the correlation processing comprises real signal wavelet transform.

**8.** The method of any one of claims 1 to 7, further comprising subjecting the wavelet transformed signal to convolution processing into the signal data before the transform in step b).

**9.** The method of any one of claims 1 to 8, wherein the correlation processing comprises creating a time variation wavelet, normalization of the signal, and convolution of the normalized signal.

**10.** The method of claims 1 to 9, wherein the correlation processing comprises performing instantaneous correlation analysis.

**11.** The method of claim 1, wherein step b) comprises:

   b-1) sampling a segment of the signal;
   b-2) generating a mother wavelet from the signal;
   b-3) optionally analyzing a correlation with the signal by extending and contracting the mother wavelet; and
   b-4) repeating b-1) to b-3) until a portion necessary for analysis of the signal is analyzed.

**12.** The method of any one of claims 1 to 11, wherein the signal comprises at least one signal calculated in a frequency band of $\delta$, $\theta$, $\alpha$, $\beta$, and $\gamma$ and four electrodes.

**13.** The method of any one of claims 1 to 12, wherein the feature and the coefficient are associated so that the level of the stimulation can be differentiated in the best manner by sigmoid fitting or a multiple regression model.

**14.** A method of analyzing an object, comprising:
analyzing a reaction to the stimulation of the object using the feature and the coefficient obtained by the method of any one of claims 1 to 13.

**15.** The method of claim 14, wherein the reaction comprises pain.

**16.** An apparatus for processing a signal of an object, comprising:

   A) a signal obtaining unit for obtaining a signal from an object;
   B) a processing unit for applying cross-correlation processing to the signal using a part or all of the signal; and
   C) a calculation unit for calculating a feature of the signal and a coefficient associated with the stimulation from a result of processing obtained in B).

**17.** An apparatus for analyzing an object, comprising:

   A) a signal obtaining unit for obtaining a signal from an object;
   B) a processing unit for applying cross-correlation processing to the signal using a part or all of the signal;
   C) a calculation unit for calculating a feature of the signal and a coefficient associated with the stimulation from a result of processing obtained in B); and
   D) an analysis unit for analyzing a characteristic of the object using the feature and the coefficient.

**18.** A program for making a computer perform a method of processing a signal of an object in response to stimulation, the method comprising:

   a) obtaining a signal in response to stimulation from the object;
   b) applying cross-correlation processing to the signal using a part or all of the signal; and
   c) calculating a feature of the signal and a coefficient associated with the stimulation from a result of processing obtained in b).

**19.** A program for making a computer perform a method of analyzing an object, the method comprising:

   a) obtaining a signal in response to stimulation from the object;

b) applying cross-correlation processing to the signal using a part or all of the signal;

c) calculating a feature of the signal and a coefficient associated with the stimulation from a result of processing obtained in b); and

d) analyzing a reaction to the stimulation of the object using the feature and the coefficient.

20. A recording medium for storing a program for making a computer perform a method of processing a signal of an object in response to stimulation, the method comprising:

a) obtaining a signal in response to stimulation from the object;

b) applying cross-correlation processing to the signal using a part or all of the signal; and

c) calculating a feature of the signal and a coefficient associated with the stimulation from a result of processing obtained in b).

21. A recording medium for storing a program for making a computer perform a method of analyzing an object, the method comprising:

a) obtaining a signal in response to stimulation from the object;

b) applying cross-correlation processing to the signal using a part or all of the signal;

c) calculating a feature of the signal and a coefficient associated with the stimulation from a result of processing obtained in b); and

d) analyzing a reaction to the stimulation of the object using the feature and the coefficient.

FIG.1

Fig. 1

Stimulation

COVAS

FIG.2

Fig. 3

FIG.3

FIG.4

Fig. 4

# FIG.5

➤ Brainwave during "no stimulation" is used for MV
- Higher numerical value is detected in time frame with high correlation to MV
- Natural world has self-similarity, which breaks down when unstable

→ Detection of pain     Stimulation

EEG(REST) Wavelet transform

Time [seconds]

Correlation breaks down upon stimulation → Decrease in numerical value

Fig. 5

# FIG.6

**Real time wavelet analysis (proposed method)**

Fig. 6

Amplitude

Signal $f(t)$

Time

① ~ ④ are performed consecutively to analyze entire time signal

① Sample a segment

② Create mother wavelet from brainwave signal

③ Extend and contract MW

④ Correlation between signal and MW

Signal after transform $T(a, b)$

Amplitude

Extract local frequency component of signal

Time

Complete calculation of 1 segment of signal T(a, b)

EP 3 662 826 A1

## FIG.7

**Experimental paradigm (high temperature stimulation)**

Fig. 7

1. Data for levels 1 and 2 and levels 5 and 6 were used to create data for 2 values of "no pain, strong pain"
2. 3 stimulations were applied for each level
3. Each stimulation was applied for 15 seconds
4. Number of samples: strong/weak × 2 levels × 40 subjects = 160 samples
5. Features: Mean value of 20 real signal wavelet features
(5 frequency bands ($\delta$, $\theta$, $\alpha$, $\beta$, and $\gamma$) at Fz, Cz, C3, and C4))

EP 3 662 826 A1

FIG.8

# FIG.9

**Robustness indicator for feature based on modulation width (amplitude) of sigmoid function**

Magnitude of pain can be classified more clearly with greater modulation width

Modulation width (amplitude)

Weak        Moderate        Strong

Pain stimulation level

Fig. 9

# FIG.10

Comparative example of amount of modulation: examples of alpha and beta bands

Fig. 10

It can be observed from this example of 2 bands that real signal wavelet has greater modulation width

A. Real signal time variation wavelet

B. Frequency power

Alpha band

Alpha band

Beta band

Modulation width between strong and weak

Thermal stimulation intensity level

Thermal stimulation intensity level

# FIG.11

**Fig. 11**

**Comparison of amount of modulation (all frequency bands): paired t-test**

In all frequency bands, modulation width is significantly greater, or has a significantly greater tendency (gamma), for real signal wavelet than frequency power

# FIG.12

## Electrical stimulation paradigm

EP 3 662 826 A1

**Fig. 12**

Weak pain level

Strong pain level

Stimulations  1、2、3

Time change

*Three levels of stimulation intensities "weak, moderate, and strong" were included.

*Each level consisted of three stimulations, and each stimulation lasted for 15 seconds.

*Three stimulations of "strong and weak pain levels" were used for differentiation analysis

*Mean amplitude, frequency power, and real signal time variation wavelet were calculated for each stimulation

*Total of 246 samples (levels (2) × number of stimulations (3) × 41 subjects)

# FIG.13

**Electrical stimulation paradigm: feature coefficients (45 features)**

**Fig. 13**

| Without real signal wavelet |
|---|

| With real signal wavelet |
|---|

| Feature | | Partial regression coefficient | | Partial regression coefficient | | Partial regression coefficient for wavelet features | |
|---|---|---|---|---|---|---|---|
| | | Mean value | Standard deviation | Mean value | Standard deviation | Mean value | Standard deviation |
| Mean amplitude | Fz | −0.0306 | 0.0776 | 0.0124 | 0.0433 | | |
| | Cz | −0.1397 | 0.1083 | −0.1098 | 0.1040 | | |
| | C3 | 0.1041 | 0.1263 | 0.1865 | 0.1304 | | |
| | C4 | 0.0119 | 0.0477 | −0.0107 | 0.0381 | | |
| Frequency $\delta$ | Fz | −0.0842 | 0.1935 | −0.5558 | 0.4632 | −2.5392 | 1.0077 |
| | Cz | 0.4176 | 0.5771 | 0.5011 | 0.5447 | −0.0464 | 0.2918 |
| | C3 | −0.1190 | 0.3007 | −0.1835 | 0.3106 | 0.0259 | 0.3471 |
| | C4 | 0.0615 | 0.2693 | 0.0106 | 0.0640 | 3.6528 | 0.9666 |
| Frequency $\Theta$ | Fz | 0.4408 | 0.2316 | 0.2519 | 0.2008 | 0.3571 | 0.5956 |
| | Cz | 0.0740 | 0.2881 | 0.0224 | 0.1371 | −3.5916 | 1.1075 |
| | C3 | −0.0887 | 0.3538 | 0.0682 | 0.2848 | −0.0775 | 0.4343 |
| | C4 | −0.2957 | 0.4617 | −0.4515 | 0.4705 | −0.7196 | 0.8653 |
| Frequency $\alpha$ | Fz | −0.1211 | 0.2394 | −0.1098 | 0.2103 | −0.3235 | 0.5072 |
| | Cz | −0.0350 | 0.1608 | −0.0308 | 0.1174 | −0.0840 | 0.3760 |
| | C3 | 0.1543 | 0.2756 | −0.0063 | 0.1729 | −3.2487 | 0.9626 |
| | C4 | −0.9309 | 0.7453 | −1.5993 | 0.8669 | 0.4219 | 0.6873 |
| Frequency $\beta$ | Fz | −1.2619 | 0.5904 | −0.2139 | 0.3365 | −3.6958 | 1.0808 |
| | Cz | −0.3202 | 0.3568 | −0.2296 | 0.2785 | 0.0187 | 0.2500 |
| | C3 | 0.0570 | 0.1525 | 0.0218 | 0.0804 | 1.2981 | 1.0874 |
| | C4 | 0.0426 | 0.2270 | 0.1502 | 0.2552 | −5.2144 | 1.1120 |
| Frequency $\gamma$ | Fz | 2.6429 | 0.8057 | 2.6064 | 0.8131 | 1.8334 | 1.2246 |
| | Cz | −0.0014 | 0.1188 | −0.0754 | 0.2140 | 0.4084 | 0.6041 |
| | C3 | −0.1709 | 0.2826 | −0.0905 | 0.1993 | 3.7275 | 1.3441 |
| | C4 | 0.0074 | 0.0407 | −0.0004 | 0.0063 | 0.6191 | 0.6505 |

| Absolute value of feature coefficient in multiple regression model is high |
|---|

FIG.14

**Electrical stimulation paradigm: differentiation accuracy (1000 times)**      **Fig. 14**

| | |
|---|---|
| **Without real signal wavelet:** | **With real signal wavelet:** |
| 52. 5 ± 6. 1 % | 64. 1±6. 4% |
| ( ≒ 49. 8±6. 9%: Randomized) | → Differentiation accuracy improved 12% |

Differentiation accuracy (0 to 1)

Differentiation accuracy (0 to 1)

EP 3 662 826 A1

## FIG.15

**Electrical stimulation**

Weak pain

Strong pain

Level 1   Level 2   Level 3

**Fig. 15**

1. Create 2 value data for "weak pain, strong pain" using data for level 1 and level 3
2. 3 stimulations for each level
3. Apply each stimulation for 15 second
4. Number of samples: 2 levels × 3 stimulations × 41 subjects = 246 samples
5. Features (20 features each): real signal wavelet and frequency power
* Fz, Cz, C3, and C4
* δ, θ, α, β, and γ

EP 3 662 826 A1

## FIG.16

Flow of differentiation analysis (SVM-RFE)

**Fig. 16**

| Data |
|---|

Real signal wavelet or frequency power for "20 features" (standardized among individuals). Unpleasantness level was set to "2 levels (unpleasant/not unpleasant)"

Level 1: pleasant pain
Level 3: unpleasant pain

Ranking of characteristics:
20 brainwave features were ranked

Determination of differentiation algorithm:
"Leave-one-out cross validation" was performed on data using support vector machine to find combination of characteristics with highest differentiation accuracy

| Comparison of differentiation accuracy |
|---|

Differentiation accuracy of real signal wavelet and frequency power is compared

EP 3 662 826 A1

# FIG.17

**A** — <u>Real signal time variation wavelet</u>

**B** — <u>Frequency power</u>

**Fig. 17**

| Feature | | Rank | Differentiation accuracy (%) |
|---|---|---|---|
| $\alpha$ | C3 | 1 | 64. 6 |
| $\delta$ | Fz | 2 | 63. 8 |
| $\Theta$ | Fz | 3 | 58. 5 |
| $\Theta$ | Cz | 4 | 58. 9 |
| $\alpha$ | Cz | 5 | 61. 8 |
| $\alpha$ | Fz | 6 | 57. 3 |
| $\Theta$ | C3 | 7 | 57. 3 |
| $\beta$ | Fz | 8 | 58. 1 |
| $\beta$ | C4 | 9 | 64. 2 |
| $\gamma$ | Cz | 10 | 66. 7 |
| $\gamma$ | C3 | 11 | 70. 7 |
| $\delta$ | Cz | 12 | __71. 1__ ● |
| $\Theta$ | C4 | 13 | 69. 5 |
| $\gamma$ | C4 | 14 | 71. 1 |
| $\beta$ | Cz | 15 | 67. 1 |
| $\alpha$ | C4 | 16 | 67. 9 |
| $\beta$ | C3 | 17 | 66. 3 |
| $\delta$ | C3 | 18 | 64. 2 |
| $\delta$ | C4 | 19 | 65. 9 |
| $\gamma$ | Fz | 20 | 66. 3 |

| Feature | | Rank | Differentiation accuracy (%) |
|---|---|---|---|
| $\delta$ | Cz | 1 | 59. 3 |
| $\gamma$ | Fz | 2 | 58. 9 |
| $\beta$ | Cz | 3 | 58. 1 |
| $\Theta$ | C3 | 4 | 60. 6 |
| $\gamma$ | Cz | 5 | 60. 2 |
| $\Theta$ | Fz | 6 | 58. 1 |
| $\alpha$ | Cz | 7 | 60. 2 |
| $\Theta$ | Cz | 8 | 56. 1 |
| $\alpha$ | Fz | 9 | 55. 3 |
| $\delta$ | Fz | 10 | 55. 3 |
| $\beta$ | C3 | 11 | 54. 5 |
| $\delta$ | C4 | 12 | 56. 1 |
| $\alpha$ | C4 | 13 | 55. 7 |
| $\gamma$ | C3 | 14 | 60. 6 |
| $\Theta$ | C4 | 15 | __62. 6__ ● |
| $\beta$ | C4 | 16 | 61. 0 |
| $\delta$ | C3 | 17 | 58. 9 |
| $\alpha$ | C3 | 18 | 57. 3 |
| $\gamma$ | C4 | 19 | 60. 2 |
| $\beta$ | Fz | 20 | 62. 2 |

Real signal wavelet (71.1%) > frequency power (62.6%)

EP 3 662 826 A1

FIG.18

Fig. 18

FIG.19

Apparatus 1000

Fig. 19

```
┌─────────────────────────┐
│ 100                     │
│ Signal obtaining unit   │
└─────────────────────────┘

┌─────────────────────────┐        ┌─────────────────────────┐
│ 200                     │        │ 300                     │
│ Processing unit         │        │ Calculation unit        │
└─────────────────────────┘        └─────────────────────────┘

┌─────────────────────────┐
│ 600                     │
│ Storage unit            │
└─────────────────────────┘

        ┌─────────────────────────┐    ┌─────────────────────────┐
        │ 400                     │    │ 500                     │
        │ Display unit            │    │ Differentiation unit    │
        └─────────────────────────┘    └─────────────────────────┘
```

## INTERNATIONAL SEARCH REPORT

| | International application No. |
|---|---|
| | PCT/JP2018/028491 |

**A. CLASSIFICATION OF SUBJECT MATTER**
Int.Cl. A61B5/0476(2006.01)i, A61B5/0484(2006.01)i, A61B10/00(2006.01)i

According to International Patent Classification (IPC) or to both national classification and IPC

**B. FIELDS SEARCHED**

Minimum documentation searched (classification system followed by classification symbols)
Int.Cl. A61B5/0476, A61B5/0484, A61B10/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII), PubMed

**C. DOCUMENTS CONSIDERED TO BE RELEVANT**

| Category* | Citation of document, with indication, where appropriate, of the relevant passages | Relevant to claim No. |
|---|---|---|
| X<br><br>Y | JP 2003-290179 A (JAPAN TOBACCO INC.) 14 October 2003, paragraphs [0014], [0021], [0025], [0038] (Family: none) | 1, 2, 4-6, 12, 14, 16-21<br>3, 7-11, 13 |
| X<br>Y | US 2003/0171685 A1 (THE JOHNS HOPKINS UNIVERSITY) 11 September 2003, abstract, paragraphs [0025], [0027]-[0030], [0034], [0038], [0074] & US 6882881 B1 & US 2005/0149123 A1 & WO 2001/028622 A2 | 1, 4-6, 12-21<br>3, 7-11, 13 |

☒ Further documents are listed in the continuation of Box C.　　☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 12 October 2018 (12.10.2018) | 23 October 2018 (23.10.2018) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2018/028491

C (Continuation).   DOCUMENTS CONSIDERED TO BE RELEVANT

| Category* | Citation of document, with indication, where appropriate, of the relevant passages | Relevant to claim No. |
|---|---|---|
| Y | 堀畑聡，実測信号 AW に適用したウェーブレットによる脳波解析，日本機械学会論文集(C 編)，2004, vol. 70, no. 694, 1795-1801, non-official translation (HORIHATA, Satoshi, "Electroencephalogram analysis of wavelet in which actual detected data is applied to AW", Transactions of the Japan Society of Mechanical Engineers. C) | 3, 7-11 |
| A | VATANKHAH, Maryam, "Pain Level Measurement Using Discrete Wavelet Transform", International Journal of Engineering and Technology, 2016, vol. 8, no. 5, 380-384 | 1-21 |
| A | HADJILEONTIADIS, Leontios J., "EEG-Based Tonic Cold Pain Characterization Using Wavelet Higher Order Spectral Features", IEEE Transactions on Biomedical Engineering, 2015, vol. 62, no. 8, 1981-1991 | 1-21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017148350 A

**Non-patent literature cited in the description**

- **N. HIRAKAWA.** Evaluation Scale of Pain. *Anesthesia 21 Century,* 2011, vol. 13 (2-40), 4-11
- **H. ARITA.** Pain VisionTM. *Anesthesia 21 Century,* 2011, vol. 13 (2-40), 11-15
- **H. FUKUYAMA, M.D., PH.D.** Tips on the functional brain imaging analysis. *The Journal of Japan Society for Cognitive Neuroscience,* 2010, vol. 12 (3
- **ARGYLE. N.** Skin Conductance Levels in Panic Disorder and Depression. *The Journal of Nervous and Mental Disease,* 1991, vol. 179, 563-566
- **MARY JO GRAP.** Pulse Oximetry. *Critical Care Nurse,* 2002, vol. 22, 69-74
- **K. TANAKA.** Perfusion Index and Pleth Variability Index. *The Journal of Japan Society for Clinical Anesthesia,* 2011, vol. 31 (2
- American Electroencephalographic Society 1991 Guidelines for standard electrode position nomenclature. *Journal of Clinical Nurophysiology,* 1991, vol. 8, 200-202
- **K. OUE ; S. ISHIMITSU.** Evaluating Emotional Responses to Sound Impressions Using Heart Rate Variability Analysis Of Heart Sound. *Tenth International Conference on Innovative Computing, Information and Control,* 2015, 118
- **PAUL S. ADDISON.** The Illustrated Wavelet Transform Handbook: Introductory Theory and Applications in Science, Engineering, Medicine and Finance. CRC Press, 15 July 2002
- **S. ISHIMITSU ; H. KOBAYASHI.** Study on Instantaneous Correlation Analyses of Acceleration Car Interior Noise using Wavelets and its Subjective Evaluation. *Transactions of the Japan Society of Mechanical Engineers,* 2006, vol. 72 (719), 2094-2100
- **H. ISHII ; H. UEMURA ; Z. ZHANG ; T. IMAMURA.** Development of identi cation for noise source using visualization of sound and vibration. *Transactions of the Society of Instrument and Control Engineers,* 2011, vol. 10 (9), 73-80
- **Z. ZHANG ; H. IKEUCHI ; H. ISHII ; H. HORIHATA ; T. IMAMURA ; T. MIYAKE.** Real-Signal Mother Wavelet and Its Application on Detection of Abnormal Signal: Designing Average Complex Real-Signal Mother Wavelet and Its Application. *Transactions of the Society of Instrument and Control Engineers,* 2011, vol. 10 (9), 73-80
- **Y. SHIMAZAKI ; A. YOSHIDA ; S. SATO ; S. NOZU.** A Study of Hyperthermia Sensitivity on Human Body based on Local Thermal Load. *Japanese society of Human-Environment System 34 in Niigata,* November 2010
- **GUYON I ; WESTON J ; BARNHILL S ; VAPNIK V.** Gene selection for cancer classification using Support Vector Machine. *Machine Learning,* 2002, vol. 46, 389-422